# EUROPEAN PATENT SPECIFICATION

(11) **EP 4 482 480 B1**
(45) Date of publication and mention of the grant of the patent: **14.10.2026**
(21) Application number: 23713227.9
(22) Date of filing: 17.02.2023
(51) Int. Cl.: A61K 31/165, A61K 31/517, A61K 31/7068, A61K 45/06, A61P 35/00, A61P 35/04

(54) **IVALTINOSTAT FOR USE IN THE TREATMENT OF PANCREATIC CANCER**
IVALTINOSTAT ZUR VERWENDUNG BEI DER BEHANDLUNG VON PANKREASKREBS
IVALTINOSTAT POUR UTILISATION DANS LE TRAITEMENT DU CANCER DU PANCRÉAS

(30) Priority: 21.02.2022 US 202263312387 P; 17.06.2022 US 202263353482 P
(43) Date of publication of application: 01.01.2025
(73) Proprietor: CG Pharmaceuticals, Inc, Orinda, CA 94563 (US)
(72) Inventor: CHO, Joong Myung, Orinda, California 94563 (US); CHO, Sangsook, Orinda, California 94563 (US)
(74) Representative: Maiwald GmbH
(86) International application number: PCT/US2023/062847
(87) International publication number: WO 2023/159200

(56) References cited:
- WO-A2-2019/043176
- ANONYMOUS: "Press Release: CG Pharmaceuticals: Progress of U.S. Phase 1b/2 Metastatic Pancreatic Cancer Clinical Trial at the ASCO Gastrointestinal Symposium", BIOSPACE, 23 January 2025 (2025-01-23), pages 1 - 3, Retrieved from the Internet <URL:https://www.biospace.com/press-releases/cg-pharmaceuticals-progress-of-u-s-phase-1b-2-metastatic-pancreatic-cancer-clinical-trial-at-the-asco-gastrointestinal-symposium>
- LEE HEE SEUNG ET AL: "A novel HDAC inhibitor, CG200745, inhibits pancreatic cancer cell growth and overcomes gemcitabine resistance", SCIENTIFIC REPORTS, vol. 7, no. 1, 30 January 2017 (2017-01-30), pages 1 - 9, XP093053276, Retrieved from the Internet <URL:https://www.nature.com/articles/srep41615> DOI: 10.1038/srep41615
- KIM KYU-PYO ET AL: "First-in-human study of the toxicity, pharmacokinetics, and pharmacodynamics of CG200745, a pan-HDAC inhibitor, in patients with refractory solid malignancies", INVESTIGATIONAL NEW DRUGS, SPRINGER US, NEW YORK, vol. 33, no. 5, 17 June 2015 (2015-06-17), pages 1048 - 1057, XP035547412, ISSN: 0167-6997, [retrieved on 20150617], DOI: 10.1007/S10637-015-0262-2
- Y. S. NA ET AL: "Effects of the HDAC inhibitor CG2 in combination with irinotecan, 5-fluorouracil, or oxaliplatin on HCT116 colon cancer cells and xenografts", ONCOLOGY REPORTS, vol. 24, no. 6, 26 October 2010 (2010-10-26), pages 1509 - 1514, XP055638817, ISSN: 1021-335X, DOI: 10.3892/or_00001012
- PAOLO PIACENTINI ET AL: "Trichostatin A enhances the response of chemotherapeutic agents in inhibiting pancreatic cancer cell proliferation", VIRCHOWS ARCHIV, SPRINGER, BERLIN, DE, vol. 448, no. 6, 28 March 2006 (2006-03-28), pages 797 - 804, XP019427639, ISSN: 1432-2307, DOI: 10.1007/S00428-006-0173-X
- HAJATDOOST LEVA ET AL: "Chemotherapy in Pancreatic Cancer: A Systematic Review", MEDICINA, vol. 54, no. 3, 11 July 2018 (2018-07-11), pages 1 - 17, XP093054166, DOI: 10.3390/medicina54030048
- JO JUNG HYUN ET AL: "A phase I/II study of ivaltinostat combined with gemcitabine and erlotinib in patients with untreated locally advanced or metastatic pancreatic adenocarcinoma", INTERNATIONAL JOURNAL OF CANCER, vol. 151, no. 9, 3 June 2022 (2022-06-03), US, pages 1565 - 1577, XP093053277, ISSN: 0020-7136, Retrieved from the Internet <URL:https://onlinelibrary.wiley.com/doi/full-xml/10.1002/ijc.34144> DOI: 10.1002/ijc.34144

## Description

### BACKGROUND

Pancreatic ductal adenocarcinoma (PDAC) is currently the third-leading cause of cancer-related mortality in the US and is expected to rise to the second-leading cause by 2030 (Siegel RL, et al., Cancer statistics, 2021, CA Cancer J Clin 71:7-33). Almost 90% of patients are diagnosed with inoperable disease (Ryan DP et al., Pancreatic Adenocarcinoma. N Engl J Med 2014; 371:1039-1049) and most have distant metastases at presentation, for which the current expected 5-year survival is only 3%.

The mainstay of treatment for metastatic PDAC is cytotoxic chemotherapy. For patients who can tolerate aggressive treatment (e.g., ECOG Performance Status 0-1), Phase 3 data support FOLFIRINOX and gemcitabine with nanoparticle albumin-bound paclitaxel (nab-paclitaxel) as the most effective first line regimens. In the PRODIGE 4/ACCORD 11 trial (Conroy T et al., FOLFIRINOX versus gemcitabine for metastatic pancreatic cancer, N Engl J Med 2011; 364:1817-1825), FOLFIRINOX produced median progression-free survival (PFS) of 6.4 months and overall survival (OS) 11.1 months. In the MPACT trial (Von Hoff DD et al., Increased survival in pancreatic cancer with nab-paclitaxel plus gemcitabine, N Engl J Med 2013; 369:1691-1703), gemcitabine/nab-paclitaxel produced median PFS 5.5 months and median OS 8.5 months. Both regimens, in their respective trials, statistically significantly improved survival compared to gemcitabine monotherapy, the prior standard of care.

First line chemotherapy for metastatic PDAC is generally continued until disease progression or development of intolerable adverse effects, with patients often reaching a plateau in response after 4-6 months. For patients with responsive or stable disease after first line therapy, the appropriate maintenance treatment strategy is undefined.

The only reported Phase 3 study to explore PDAC maintenance therapy, the POLO trial, showed that the PARP inhibitor olaparib improved PFS compared to placebo among patients with germline BRCA1/2 (gBRCA) mutations after ≥16 weeks of platinum-based therapy (median PFS 7.4 vs. 3.8 months; HR 0.53, p=0.004) (Golan T et al., Maintenance olaparib for germline BRCA-mutated metastatic pancreatic cancer, N Engl J Med, 2019 25;381(4):317-327). However, the use of placebo as a control arm does not accurately represent current clinical practice. Moreover, only 1-2% of individuals with PDAC harbor gBRCA mutations (Shindo K et al., Deleterious Germline Mutations in Patients With Apparently Sporadic Pancreatic Adenocarcinoma, J Clin Oncol. 2017 Oct 20;35(30):3382-3390) leaving unresolved the question of the optimal maintenance approach for non-biomarker-selected patients.

Maintenance therapy should ideally delay disease progression and preserve disease-related quality of life by minimizing cumulative toxicity and maximizing convenience. In the absence of Phase 3 data to guide this decision, efficacy and toxicity are often balanced by reducing the number of first line chemotherapeutic agents (Hammel P et al., Maintenance therapies in metastatic pancreatic cancer: present and future with a focus on PARP inhibitors, Ther Adv Med Oncol. 2020;12:1758835920937949; Petrioli R et al., Gemcitabine plus nab-paclitaxel followed by maintenance treatment with gemcitabine alone as first-line treatment for older adults with locally advanced or metastatic pancreatic cancer, J Geriatr Oncol. 2020 May; 11(4):647-651; Relias V, Maloney A, Smith MH, et al. Does "OPTINAB" strategy ("stop-and-go") work in treatment of advanced pancreatic cancer (APC) with nab-paclitaxel-gemcitabine? Cancer Chemother Pharmacol. 2017 Aug; 80(2):371-375). While there exist data support maintenance with the FOLFIRI doublet, further reduction to fluoropyrimidine monotherapy could avoid irinotecan-associated toxicities. Prospective studies are needed to better quantify the efficacy, toxicity, and quality of life impact of these options. Without definitive data, current National Comprehensive Cancer Network (NCCN) guidelines list FOLFIRI, FOLFOX (oxaliplatin, leucovorin, and 5-FU), or the oral fluoropyrimidine capecitabine as possible post-FOLFIRINOX maintenance therapies (National Comprehensive Cancer Network Guidelines Pancreatic Adenocarcinoma. NCCN.org. Version 2. Feb 25, 2021).

Lee, Seung Hee et al. reported that CG200745 significantly improved pancreatic cancer sensitivity to gemcitabine, with a prominent antitumor effect on gemcitabine-resistant pancreatic cancer cells (Lee, Hee Seung, et al. "A novel HDAC inhibitor, CG200745, inhibits pancreatic cancer cell growth and overcomes gemcitabine resistance." Scientific reports 7.1 (2017): 41615).

While de-escalation of first line regimens represents an intuitive approach, introducing novel agents with different mechanisms of action could reduce the risk of chemotherapeutic cross-resistance and prolong disease control. There exists a need to develop new therapies for the treatment or maintenance of pancreatic cancer (e.g., pancreatic adenocarcinoma, locally advanced pancreatic adenocarcinoma, or metastatic pancreatic adenocarcinoma), in particular metastatic PDAC.

### SUMMARY

The references to methods of treatment by therapy of this description are to be interpreted as references to compounds, pharmaceutical compositions and medicaments of the present invention for use in those methods. The invention is set out in the appended set of claims and relates to ivaltinostat for use in a method of treating pancreatic cancer in a subject in need thereof, wherein the subject has been treated with a first line fluoropyrimidine-based chemotherapy, wherein ivaltinostat is for use in combination with one or more additional anticancer agents, wherein the one or more additional anti-cancer agents is capecitabine.

In some embodiments, the effective amount of ivaltinostat is about 10 mg/m² to about 750 mg/m². In some embodiments, the effective amount of ivaltinostat is about 25 mg/m² to about 500 mg/m². In further embodiments, the effective amount of ivaltinostat is about 50 mg/m² to about 375 mg/m². In further embodiments, the effective amount of ivaltinostat is about 60 mg/m², about 125 mg/m² or about 250 mg/m². In some embodiments, ivaltinostat is for administration by intravenous infusion. In further embodiments, the intravenous infusion is over about 30 minutes to about 120 minutes. In still further embodiments, the intravenous infusion is about 50 to 70 minutes, or about 60 minutes. In some embodiments, ivaltinostat is for administration on day 1 and day 8 of a 21-day cycle. In other embodiments, ivaltinostat is for oral administration. In some such embodiments, ivaltinostat is for oral administration from about 10 mg to about 1000 mg, about 20 mg to about 500 mg, about 30 mg to about 400 mg, or about 50 mg to about 300 mg. In some further embodiments, the oral administration is once, twice or three times a day. In further embodiments, ivaltinostat is for oral administration from about 10 mg/day to about 1000 mg/day, about 20 mg/day to about 500 mg/day, about 30 mg/day to about 400 mg/day, or about 50 mg/day to about 300 mg/day. In further embodiments, ivaltinostat is for oral administration about 50 mg/day, about 100 mg/day, about 200 mg/day, or about 300 mg/day. In further embodiments, the effective amount of capecitabine is from about 200 mg/m² to about 2000 mg/m², from about 500 mg to about 1500 mg/m², or from about 750 mg/m² to about 1250 mg/m². In still further embodiments, the effective amount of capecitabine is about 1000 mg/m². In some embodiments, capecitabine is for oral administration. In further embodiments, capecitabine is for administration twice daily, for example for 2 weeks (e.g., in a 21 day cycle).

In some embodiments, the pancreatic cancer is pancreatic adenocarcinoma, locally advanced pancreatic adenocarcinoma, or metastatic pancreatic adenocarcinoma, also referred to as pancreatic ductal pancreatic ductal adenocarcinoma (PDAC). The subject has been treated with a first line fluoropyrimidine-based chemotherapy, such as a FOLFIRINOX chemotherapy regimen (an abbreviation for a chemotherapy combination used to treat pancreatic cancer that has spread to other parts of the body. It may include but is not limited to the drugs leucovorin calcium, fluorouracil, irinotecan hydrochloride, and oxaliplatin). In further embodiments, the subject has been treated with a FOLFIRINOX chemotherapy regimen. In still further embodiments, the subject has metastatic pancreatic adenocarcinoma and the subject has a response or stability with the first line fluoropyrimidine-based chemotherapy. In still further embodiments, the subject has metastatic pancreatic adenocarcinoma whose disease has not progressed on a first line fluoropyrimidine-based chemotherapy.

Another aspect of the present disclosure relates to a method of using or use of an effective amount of ivaltinostat for treating or ameliorating pancreatic cancer (e.g., pancreatic adenocarcinoma, locally advanced pancreatic adenocarcinoma, or metastatic pancreatic carcinoma, wherein the subject's pancreatic cancer has not progressed on a first line fluoropyrimidine-based chemotherapy, and wherein the effective amount of ivaltinostat is for use in combination with an effective amount of one or more anti-cancer agents, wherein the one or more additional anti-cancer agents is capecitabine. In some embodiments, the method includes selecting or identifying a subject with metastatic pancreatic adenocarcinoma whose disease has not progressed on a first line fluoropyrimidine-based chemotherapy and administering an effective amount of ivaltinostat in combination with an effective amount of one or more anti-cancer agents to the subject. In some embodiments, the fluoropyrimidine-based chemotherapy can include oxaliplatin, irinotecan, leucovorin, or 5-fluorouracil, or combinations thereof. In some embodiments, the one or more anti-cancer agents can include the fluoropyrimidine-based chemotherapy agents or capecitabine, or combinations thereof. In some embodiments, the effective amount of ivaltinostat is from about 50 mg/m² to about 375 mg/m², about 60 mg/m², about 125 mg/m² or about 250 mg/m². In some embodiments, ivaltinostat is for administration by intravenous infusion. In further embodiments, the intravenous infusion is over about 30 minutes to about 120 minutes, or about 50 to 70 minutes, or about 60 minutes. In some embodiments, ivaltinostat is for administration on day 1 and day 8 of a 21-day cycle. In other embodiments, ivaltinostat is for oral administration. In some such embodiments, ivaltinostat is for oral administration from about 10 mg to about 1000 mg, about 20 mg to about 500 mg, about 30 mg to about 400 mg, or about 50 mg to about 300 mg. In some further embodiments, the oral administration is once, twice or three times a day. In further embodiments, ivaltinostat is for oral administration from about 10 mg/day to about 1000 mg/day, about 20 mg/day to about 500 mg/day, about 30 mg/day to about 400 mg/day, or about 50 mg/day to about 300 mg/day. In further embodiments, ivaltinostat is for oral administration about 50 mg/day, about 100 mg/day, about 200 mg/day, or about 300 mg/day. In some embodiments, the one or more additional anti-cancer agents can include or is capecitabine. In further embodiments, the effective amount of capecitabine is from about 200 mg/m² to about 2000 mg/m², from about 500 mg to about 1500 mg/m², or from about 750 mg/m² to about 1250 mg/m², or about 1000 mg/m². In still further embodiments, the effective amount of capecitabine is about 1000 mg/m². In some embodiments, capecitabine is for oral administration, for example twice daily, or twice daily for 2 weeks (e.g., in a 21 day cycle).

In any embodiments of the method of using or use of an effective amount of ivaltinostat for treating cancer in a subject in need thereof described herein, the administration of ivaltinostat is discontinued if the subject's absolute neutrophil count (ANC) is less than about 1,000/µL or the platelet count is less than about 100,000/µL. In some embodiments, if the subject suffers from one or more hematologic adverse events related to ivaltinostat administration, the amount of ivaltinostat is reduced after the subject recovers from the one or more hematologic adverse events. In some such embodiments, the hematologic adverse events can include Grade 3 neutropenia or Grade 3 thrombocytopenia. In some further embodiments, the amount of ivaltinostat is reduced to about 80% when a second episode of Grade 3 neutropenia or Grade 3 thrombocytopenia occurs. In further embodiments, ivaltinostat is discontinued when a third episode of Grade 3 neutropenia or Grade 3 thrombocytopenia occurs. In other embodiments, the hematologic adverse events include Grade 4 neutropenia, Grade 4 thrombocytopenia, or Grade 3 febrile neutropenia. In further embodiments, the amount of ivaltinostat is reduced to about 80% when a first episode of Grade 4 neutropenia, Grade 4 thrombocytopenia, or Grade 3 febrile neutropenia occurs. In further embodiments, the amount of ivaltinostat is reduced to about 64% when a second episode of Grade 4 neutropenia, Grade 4 thrombocytopenia, or Grade 3 febrile neutropenia occurs. In still further embodiments, ivaltinostat is discontinued when a third episode of Grade 4 neutropenia, Grade 4 thrombocytopenia, or Grade 3 febrile neutropenia occurs. In other embodiments, the hematologic adverse events include Grade 4 febrile neutropenia. In further embodiments, the amount of ivaltinostat is reduced to about 80% when a first episode of Grade 4 febrile neutropenia occurs. In further embodiments, ivaltinostat is discontinued when a second episode of Grade 4 febrile neutropenia occurs.

In any embodiments of the method or use described herein, ivaltinostat is for administration as a combination therapy with one or more additional anti-cancer agents, wherein the one or more additional anti-cancer agents is capecitabine. In further embodiments, the ivaltinostat combination therapy is for use in providing maintenance treatment for pancreatic cancer (e.g., advanced or metastatic pancreatic adenocarcinoma). Ivaltinostat may be for intravenous infusion (IV) or oral administration. In some embodiments, ivaltinostat is for oral administration when a subject is in a fast state. In other embodiments, ivaltinostat is for oral administration when a subject is in a fed state.

An additional aspect of the present disclosure relates to a pharmaceutical composition comprising an effective amount of ivaltinostat for use in treating or ameliorating pancreatic adenocarcinoma in a subject in need thereof, wherein the subject's pancreatic adenocarcinoma has not progressed on a first line fluoropyrimidine-based chemotherapy, and wherein the pharmaceutical composition is for use in combination with an effective amount of one or more anti-cancer agents, wherein the one or more additional anti-cancer agents is capecitabine.

### BRIEF DESCRIPTION OF THE DRAWING

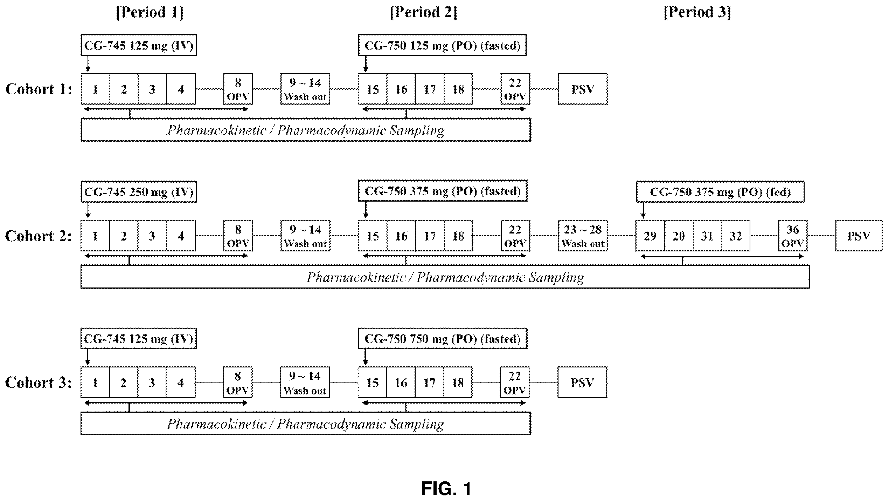

**FIG. 1** illustrates a single dose ivaltinostat Phase 1 clinical study design as described in Example 3.

### DETAILED DESCRIPTION

Ivaltinostat (E)-N1-(3-(dimethylamino)propyl)-N8-hydroxy-2-((naphthalen-1-yloxy)methyl)oct-2-enediamide phosphate is a novel anticancer therapeutic candidate that inhibits enzymatic activity of histone deacetylase (HDAC).

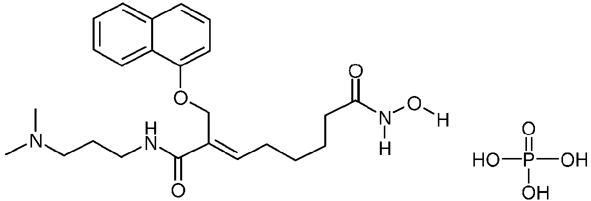

Histone acetylation/deacetylation is an integral component of epigenetic regulation mechanisms in human cells. This process is mediated by counterbalanced enzymes: histone acetyltransferase (HAT), which adds acetyl groups to, and histone deacetylase (HDAC), which removes the acetyl groups from lysine residues present at the N-terminus of histones and other proteins. Acetylation neutralizes the positive charge on histones, unwrapping DNA from the nucleosome, opening chromatin, and leading to increased gene transcription. The balance between HDAC and HAT activity can modulate gene expression in response to changes in the molecular environment.

Essential processes such as cell proliferation, migration, angiogenesis, DNA damage repair, and death depend on coordinated HDAC/HAT activity, histone modification, nucleosome remodeling, and gene transcription. Aberrant epigenetic regulation due to HDAC overactivity can cause tumor suppressor gene silencing and neoplastic cell transformation. Indeed, HDAC overexpression has been detected in wide range of cancers.

The reversible nature of epigenetic modification creates a therapeutic opportunity to restore normal gene expression in malignant cells by inhibiting HDAC activity. In pre-clinical studies, HDAC inhibition has been shown to limit tumor growth and angiogenesis, control tumor differentiation, and induce DNA damage, cell cycle arrest, apoptosis and autophagy. HDAC inhibitors (HDACi) sensitize tumor cells to chemotherapy and radiation and can influence anti-tumoral immune responses to overcome immunotherapeutic resistance.

HDAC enzymes are overexpressed in PDAC cells and are implicated in oncogenesis as well as tumor progression. In preclinical studies, HDAC were found to regulate PDAC cell mobility/migration and confer chemotherapeutic resistance. In humans, HDAC expression in PDAC surgical resection specimens correlates with worse prognosis. HDAC inhibition thus represents an appealing therapeutic approach, either as monotherapy or as an adjunct to established treatments.

After first line fluoropyrimidine-based therapy, most commonly oxaliplatin, irinotecan, leucovorin, and 5-fluorouracil (FOLFIRINOX), the optimal maintenance treatment for advanced pancreatic adenocarcinoma is undefined. Simplifying a multi-agent regimen to fluoropyrimidine monotherapy is a common practice for maintenance treatment, and the oral fluoropyrimidine, capecitabine, is commonly used to maximize convenience. Capecitabine is labeled for monotherapy for the treatment of adjuvant Dukes' C colon cancer and metastatic colon cancer at a recommended dose of 1250 mg/m² twice daily for 2 weeks followed by a 1-week rest. This dose schedule was effective and tolerable in the first line setting for patients with pancreatic cancer (Cartwright, J Clin Oncol 2002 Jan 1; 20(1):160-1642002).

Other useful pharmaceutical salt of ivaltinostat free base (E)-N1-(3-(dimethylamino)propyl)-N8-hydroxy-2-((naphthalen-1-yloxy)methyl)oct-2-enediamide include other acid addition salts, such as tartaric acid addition salt, which is disclosed in WO 2018/230829 and U.S. Publication No. 2020/0283371 A1. In addition to the phosphate salt, other pharmaceutically acceptable salt of ivaltinostat free base may also be used in the methods described herein.

### Definitions

The section headings used herein are for organizational purposes only and are not to be construed as limiting the subject matter described.

Unless defined otherwise, all technical and scientific terms used herein have the same meaning as is commonly understood by one of ordinary skill in the art. The use of the term "including" as well as other forms, such as "include," "includes," and "included," is not limiting. The use of the term "having" as well as other forms, such as "have," "has," and "had," is not limiting. As used in this specification, whether in a transitional phrase or in the body of the claim, the terms "comprise(s)" and "comprising" are to be interpreted as having an open-ended meaning. That is, the above terms are to be interpreted synonymously with the phrases "having at least" or "including at least." For example, when used in the context of a process, the term "comprising" means that the process includes at least the recited steps but may include additional steps. When used in the context of a compound, composition, formulation, or device, the term "comprising" means that the compound, composition, formulation, or device includes at least the recited features or components, but may also include additional features or components.

The terms "effective amount" and "therapeutically effective amount" are broad terms and are to be given their ordinary and customary meaning to a person of ordinary skill in the art (and are not to be limited to a special or customized meaning), and refer without limitation to a sufficient amount of an agent or a compound being administered which will relieve to some extent one or more of the symptoms of the disease or condition being treated. The result can be reduction and/or alleviation of the signs, symptoms, or causes of a disease, or any other desired alteration of a biological system. For example, an "effective amount" for therapeutic uses is the amount of the composition comprising a compound as disclosed herein required to provide a clinically significant decrease in disease symptoms. An appropriate "effective" amount in any individual case may be determined using techniques, such as a dose escalation study. Where a drug has been approved by the U.S. Food and Drug Administration (FDA) or a counterpart foreign medicines agency, a "therapeutically effective amount" optionally refers to the dosage approved by the FDA or its counterpart foreign agency for treatment of the identified disease or condition.

"Treat," "treatment," or "treating," as used herein refers to administering a pharmaceutical composition/formulation for prophylactic and/or therapeutic purposes. The term "prophylactic treatment" refers to treating a patient who is not yet suffering from a disease, but who is susceptible to, or otherwise at risk of, a particular disease, whereby the treatment reduces the likelihood that the patient will develop a disease. The term "therapeutic treatment" refers to administering treatment to a patient already suffering from a disease.

The terms "co-administration" and similar terms as used herein are broad terms, and are to be given their ordinary and customary meaning to a person of ordinary skill in the art (and are not to be limited to a special or customized meaning), and refer without limitation to administration of the selected therapeutic agents to a single patient, and are intended to include treatment regimens in which the agents are administered by the same or different route of administration or at the same or different time.

The term "pharmaceutically acceptable salt" as used herein is a broad term and is to be given its ordinary and customary meaning to a person of ordinary skill in the art (and is not to be limited to a special or customized meaning), and refers without limitation to a salt of a compound that does not cause significant irritation to an organism to which it is administered and does not abrogate the biological activity and properties of the compound. In some embodiments, the salt is an acid addition salt of the compound. Pharmaceutical salts can be obtained by reacting a compound with inorganic acids such as hydrohalic acid (*e.g*., hydrochloric acid or hydrobromic acid), sulfuric acid, nitric acid, and phosphoric acid. Pharmaceutical salts can also be obtained by reacting a compound with an organic acid such as aliphatic or aromatic carboxylic or sulfonic acids, for example formic acid, acetic acid (AcOH), propionic acid, glycolic acid, pyruvic acid, malonic acid, maleic acid, fumaric acid, trifluoroacetic acid (TFA), benzoic acid, cinnamic acid, mandelic acid, succinic acid, lactic acid, malic acid, tartaric acid, citric acid, ascorbic acid, nicotinic acid, methanesulfonic acid, ethanesulfonic acid, p-toluensulfonic acid, salicylic acid, stearic acid, muconic acid, butyric acid, phenylacetic acid, phenylbutyric acid, valproic acid, 1,2-ethanedisulfonic acid, 2-hydroxyethanesulfonic acid, benzenesulfonic acid, 2-naphthalenesulfonic acid, or naphthalenesulfonic acid. Pharmaceutical salts can also be obtained by reacting a compound with a base to form a salt such as an ammonium salt, an alkali metal salt, such as a lithium, sodium or a potassium salt, an alkaline earth metal salt, such as a calcium, magnesium or aluminum salt, a salt of organic bases such as dicyclohexylamine, N-methyl-D-glucamine, tris(hydroxymethyl)methylamine, (C₁-C₇ alkyl)amine, cyclohexylamine, dicyclohexylamine, triethanolamine, ethylenediamine, ethanolamine, diethanolamine, triethanolamine, tromethamine, and salts with amino acids such as arginine and lysine; or a salt of an inorganic base, such as aluminum hydroxide, calcium hydroxide, potassium hydroxide, sodium carbonate, sodium hydroxide, or the like.

It is understood that the compounds described herein can be labeled isotopically or by another other means, including, but not limited to, the use of chromophores or fluorescent moieties, bioluminescent labels, or chemiluminescent labels. Substitution with isotopes such as deuterium may afford certain therapeutic advantages resulting from greater metabolic stability, such as, for example, increased *in vivo* half-life or reduced dosage requirements. Each chemical element as represented in a compound structure may include any isotope of said element. For example, in a compound structure a hydrogen atom may be explicitly disclosed or understood to be present in the compound. At any position of the compound that a hydrogen atom may be present, the hydrogen atom can be any isotope of hydrogen, including but not limited to hydrogen-1 (protium), hydrogen-2 (deuterium), and hydrogen-3 (tritium). Thus, reference herein to a compound encompasses all potential isotopic forms unless the context clearly dictates otherwise.

It is understood that the methods and formulations described herein include the use of pharmaceutically acceptable salts and/or conformers of compounds of preferred embodiments, as well as metabolites and active metabolites of these compounds having the same type of activity. A conformer is a structure that is a conformational isomer. Conformational isomerism is the phenomenon of molecules with the same structural formula but different conformations (conformers) of atoms about a rotating bond.

Ivaltinostat as described in the Examples in connection with the various clinical studies, refers to a preferred embodiment of the pharmaceutical salt of ivaltinostat, i.e., its phosphate salt (E)-N1-(3-(dimethylamino)propyl)-N8-hydroxy-2-((naphthalen-1-yloxy)methyl)oct-2-enediamide) phosphate. One skilled in the art understands that other pharmaceutical salts of ivaltinostat as described herein, including but not limited to tartaric acid addition salt, may also be used in the cancer treatment described herein.

### Methods of Treating Cancer

The invention is set out in the appended set of claims and relates to ivaltinostat for use in a method of treating pancreatic cancer in a subject in need thereof, wherein the subject has been treated with a first line fluoropyrimidine-based chemotherapy, wherein ivaltinostat is for use in combination with one or more additional anti-cancer agents, wherein the one or more additional anti-cancer agents is capecitabine.

In some embodiments of the method described herein, the effective amount of ivaltinostat is about 10 mg/m² to about 750 mg/m², for example, about 10, 15, 20, 25, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190, 195, 200, 205, 210, 215, 220, 225, 230, 235, 240, 245, 250, 255, 260, 265, 270, 275, 280, 285, 290, 295, 300, 325, 350, 375, 400, 425, 450, 475, 500, 525, 550, 575, 600, 625, 650, 675, 700, 725, or 750 mg/m², or a range defined by any two of the preceding values. In some further embodiments, the effective amount of ivaltinostat is about 25 mg/m² to about 500 mg/m². In further embodiments, the effective amount of ivaltinostat is about 50 mg/m² to about 375 mg/m². In further embodiments, the effective amount of ivaltinostat is about 60 mg/m², about 125 mg/m² or about 250 mg/m². In some other embodiments, the effective amount of ivaltinostat is about 10 mg to about 750 mg, for example, about 10, 15, 20, 25, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190, 195, 200, 205, 210, 215, 220, 225, 230, 235, 240, 245, 250, 255, 260, 265, 270, 275, 280, 285, 290, 295, 300, 325, 350, 375, 400, 425, 450, 475, 500, 525, 550, 575, 600, 625, 650, 675, 700, 725, or 750 mg, or a range defined by any two of the preceding values. In some further embodiments, the effective amount of ivaltinostat is about 25 mg to about 500 mg. In further embodiments, the effective amount of ivaltinostat is about 50 mg to about 375 mg. In further embodiments, the effective amount of ivaltinostat is about 60 mg, about 125 mg or about 250 mg. In some embodiments, ivaltinostat is administered by intravenous infusion. In further embodiments, the intravenous infusion is over about 30 minutes to about 120 minutes. In still further embodiments, the intravenous infusion is about 50 to 70 minutes, or about 60 minutes. In other embodiments, ivaltinostat is administered orally. In some embodiments, ivaltinostat is administered on day 1 and day 8 of a 21-day cycle. In other embodiments, ivaltinostat is orally administered once a day, twice a day or three times a day. In some further embodiments, the effective amount of ivaltinostat for oral administration is about 10 mg/day to about 1000 mg/day, for example, about 10, 15, 20, 25, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190, 195, 200, 205, 210, 215, 220, 225, 230, 235, 240, 245, 250, 255, 260, 265, 270, 275, 280, 285, 290, 295, 300, 325, 350, 375, 400, 425, 450, 475, 500, 525, 550, 575, 600, 625, 650, 675, 700, 725, or 750 mg/day, or a range defined by any two of the preceding values. In some further embodiments, the effective amount of ivaltinostat for oral administration is about 50 mg/day, about 100 mg/day, about 200 mg/day, or about 300 mg/day.

The method described herein comprises administering an effective amount of one or more additional anti-cancer agents, wherein said additional anti-cancer agent is capecitabine. In further embodiments, the effective amount of capecitabine is from about 200 mg/m² to about 2000 mg/m², for example, about 200, 225, 250, 275, 300, 325, 350, 375, 400, 425, 450, 475, 500, 525, 550, 575, 600, 625, 650, 675, 700, 725, 750, 775, 800, 825, 850, 875, 900, 925, 950, 975, 1000, 1050, 1100, 1150, 1200, 1250, 1300, 1350, 1400, 1450, 1500, 1550, 1600, 1650, 1700, 1750, 1800, 1850, 1900, 1950, or 2000 mg/m², or a range defined by any two of the preceding values. In still further embodiments, the effective amount of capecitabine is about 1000 mg/m². In some embodiments, capecitabine is administered orally. In further embodiments, capecitabine is administered twice daily, for example for 2 weeks (e.g., in a 21 day cycle).

In further embodiments, the pancreatic cancer comprises or is metastatic pancreatic adenocarcinoma, also referred to as pancreatic ductal pancreatic ductal adenocarcinoma (PDAC). In further embodiments, the subject has been treated with FOLFIRINOX chemotherapy regimen (an abbreviation for a chemotherapy combination used to treat pancreatic cancer that has spread to other parts of the body. It may include but not limited to the drugs leucovorin calcium, fluorouracil, irinotecan hydrochloride, and oxaliplatin). In further embodiments, the method comprising selecting or identifying a subject who has been treated with a FOLFIRINOX chemotherapy regimen (e.g., oxaliplatin, irinotecan, leucovorin, or 5-fluorouracil, or pharmaceutically acceptable salts and combinations thereof). In still further embodiments, the method comprising selecting or identifying a subject with metastatic pancreatic adenocarcinoma after the subject has a response or stability with a FOLFIRINOX chemotherapy regimen. In still further embodiments, the method comprising selecting or identifying patients with metastatic pancreatic adenocarcinoma whose disease has not progressed on a first line fluoropyrimidine-based chemotherapy.

### Patient Subpopulation Selection

Another aspect of the present disclosure relates to a method of treating or ameliorating pancreatic cancer (e.g., pancreatic adenocarcinoma, locally advanced or metastatic pancreatic adenocarcinoma) in a subject in need thereof, comprising selecting or identifying a subject with metastatic pancreatic adenocarcinoma whose disease has not progressed on a first line fluoropyrimidine-based chemotherapy and administering an effective amount of ivaltinostat in combination with an effective amount of one or more anti-cancer agents to the subject, wherein the one or more additional anti-cancer agents is capecitabine. In some embodiments, the fluoropyrimidine-based chemotherapy comprises oxaliplatin, irinotecan, leucovorin, or 5-fluorouracil, or combinations thereof. In some embodiments, the one or more anti-cancer agents comprises the fluoropyrimidine-based chemotherapy agents or capecitabine, or combinations thereof. In some embodiments, the effective amount of ivaltinostat is from about 50 mg/m² to about 375 mg/m², about 60 mg/m², about 125 mg/m² or about 250 mg/m². In some embodiments, ivaltinostat is administered by intravenous infusion. In further embodiments, the intravenous infusion is over about 30 minutes to about 120 minutes, or about 50 to 70 minutes, or about 60 minutes. In other embodiments, ivaltinostat is administered orally. In some embodiments, ivaltinostat is administered on day 1 and day 8 of a 21-day cycle. In some embodiments, the one or more additional anti-cancer agents comprises or is capecitabine. In further embodiments, the effective amount of capecitabine is from about 200 mg/m² to about 2000 mg/m², from about 500 mg to about 1500 mg/m², or from about 750 mg/m² to about 1250 mg/m², or about 1000 mg/m². In still further embodiments, the effective amount of capecitabine is about 1000 mg/m². In some embodiments, capecitabine is administered orally, for example administered twice daily for 2 weeks (e.g., in a 21 day cycle).

### Drug Interactions

Nonclinical studies demonstrate that ivaltinostat is a substrate for CYP3A and CYP2D6. Concomitant use of drugs that are moderate or strong inhibitors or inducers of these CYP enzymes should be avoided. In addition, in in vitro studies, ivaltinostat inhibited CYP2D6 with an IC50 of 5.5 µM; IC50 values were > 10 µM for CYPs 1A2 and 3A4 and > 90 µM for CYPs 2C9 and 2C19. Concomitant use of drugs that are substrates of CYP2D6 and have a narrow therapeutic index is not advised.

Another aspect of the present disclosure relates to a method of providing ivaltinostat therapy to a subject in need thereof, comprising administering an effective amount of ivaltinostat to the subject; and advising the subject not to concomitantly take one or more drugs that is an inhibitor or inducer of CYP3A and/or CYP2D6 enzymes.

The ivaltinostat therapy described herein, is for treating pancreatic cancer. In further embodiments, the cancer comprises or is metastatic pancreatic adenocarcinoma, or PDAC. In further embodiments, the subject has been treated with FOLFIRINOX chemotherapy regimen (an abbreviation for a chemotherapy combination used to treat pancreatic cancer that has spread to other parts of the body. It may include but not limited to the drugs leucovorin calcium, fluorouracil, irinotecan hydrochloride, and oxaliplatin). In further embodiments, the method comprising selecting or identifying a subject who has been treated with a FOLFIRINOX chemotherapy regimen. In still further embodiments, the method comprising selecting or identifying a subject with metastatic pancreatic adenocarcinoma after the subject has a response or stability with a FOLFIRINOX chemotherapy regimen. In still further embodiments, the method comprising selecting or identifying patients with metastatic pancreatic adenocarcinoma whose disease has not progressed on a first line fluoropyrimidine-based chemotherapy. The ivaltinostat therapy is a combination therapy with one or more additional anti-cancer agents, wherein the one or more additional anti-cancer agents is capecitabine. In further embodiments, the one or more additional anti-cancer agents comprises a fluoropyrimidine-based therapeutic agent, oxaliplatin, irinotecan, leucovorin, 5-fluorouracil, capecitabine, gemcitabine, erlotinib, or combinations thereof. In one embodiment, the one or more additional anti-cancer agent comprises or is capecitabine. In some embodiments, the one or more drugs that should not be taken concomitantly with ivaltinostat comprises Alfentanil, Cyclosporine, Digoxin, Dihydroergotamine, Ergotamine, Fentanyl, Mephenytoin, Phenytoin, Pimozide, Quinidine, Sirolimus, Tacrolimus, Tizanidine, Theophylline, or Warfarin.

### Dose Adjustment

In any embodiments of the method of treating cancer or the method of providing the ivaltinostat therapy described herein, the method may further comprise measuring the subject's absolute neutrophil count (ANC) and platelet count; and discontinuing the administration if the subject's ANC is less than about 1,000/µL or the platelet count is less than 100,000/µL.

In some embodiments, the method may further comprise monitoring the subject for one or more hematologic adverse events related to ivaltinostat administration; and reducing the amount of ivaltinostat (e.g., after the subject recovers from the one or more hematologic adverse events). In some such embodiments, the hematologic adverse events comprise Grade 3 neutropenia or Grade 3 thrombocytopenia. In some further embodiments, the amount of ivaltinostat is reduced to about 80% when a second episode of Grade 3 neutropenia or Grade 3 thrombocytopenia occurs. In further embodiments, ivaltinostat is discontinued when a third episode of Grade 3 neutropenia or Grade 3 thrombocytopenia occurs. In other embodiments, the hematologic adverse events comprise Grade 4 neutropenia, Grade 4 thrombocytopenia, or Grade 3 febrile neutropenia. In further embodiments, the amount of ivaltinostat is reduced to about 80% when a first episode of Grade 4 neutropenia, Grade 4 thrombocytopenia, or Grade 3 febrile neutropenia occurs. In further embodiments, the amount of ivaltinostat is reduced to about 64% when a second episode of Grade 4 neutropenia, Grade 4 thrombocytopenia, or Grade 3 febrile neutropenia occurs. In still further embodiments, ivaltinostat is discontinued when a third episode of Grade 4 neutropenia, Grade 4 thrombocytopenia, or Grade 3 febrile neutropenia occurs. In other embodiments, the hematologic adverse events comprise Grade 4 febrile neutropenia. In further embodiments, the amount of ivaltinostat is reduced to about 80% when a first episode of Grade 4 febrile neutropenia occurs. In further embodiments, ivaltinostat is discontinued when a second episode of Grade 4 febrile neutropenia occurs. Summary on the dose modification of ivaltinostat based on one or more adverse events (e.g., hematologic adverse events, diarrhea, nausea, vomiting, or those related to liver function test) may be found in Table A, B, C, and E below.

**Table A**

| **Upon Recovery from the Following Adverse Event** | **Ivaltinostat Dose Modification** | **Capecitabine Dose Modification** |
|---|---|---|
| Gr 3 neutropenia or thrombocytopenia | 1^{st} episode = RP2D mg/m² | 1^{st} episode = 1000 mg/m² |
| | 2^{nd} episode = 80% of RP2D mg/m² | 2^{nd} episode = 800 mg/m² |
| | 3^{rd} episode = discontinue | 3^{rd} episode = discontinue |
| Gr 4 neutropenia or thrombocytopenia, or Gr 3 febrile neutropenia | 1^{st} episode = 80% of RP2D mg/m² | 1^{st} episode = 800 mg/m² |
| | 2^{nd} episode = 64% of RP2D mg/m² | 2^{nd} episode = 600 mg/m² |
| | 3^{rd} episode = discontinue | 3^{rd} episode = discontinue |
| Gr 4 febrile neutropenia | 1^{st} episode = 80% of RP2D mg/m² | 1^{st} episode = 800 mg/m² |
| | 2^{nd} episode = discontinue | |
| | | 2^{nd} episode = discontinue |

**Table B**

| **Diarrhea*** | | |
|---|---|---|
| **CTCAE v5.0 Toxicity** | **Ivaltinostat Dose Modification** | **Capecitabine Dose Modification** |
| Grade 1 | No dose adjustment | No dose adjustment |
| Grade 2 | No dose adjustment | Interrupt until Grade ≤1 |
| | | 1^{st} episode = 800 mg/m² |
| | | 2^{nd} episode = 600 mg/m² |
| | | 3^{rd} episode = discontinue |
| Grade 3 | Interrupt until Grade ≤2 | Interrupt until Grade ≤1 |
| | 1^{st} episode = 80% of RP2D mg/m² | 1^{st} episode = 800 mg/m² |
| | 2^{nd} episode = 64% of RP2D mg/m² | 2^{nd} episode = 600 mg/m² |
| | 3^{rd} episode = discontinue | 3^{rd} episode = discontinue |
| Grade 4 | Interrupt until Grade ≤2 | Interrupt until Grade ≤1 |
| | 1^{st} episode = 64% of RP2D mg/m² | 1^{st} episode = 600 mg/m² |
| | 2^{nd} episode = discontinue | 2^{nd} episode = discontinue |

| | | |
|---|---|---|
| *Medical management should first be attempted. If diarrhea persists despite optimal medical management, this table should be used to guide dose adjustments. | | |

**Table C**

| **Nausea/Vomiting*** | | |
|---|---|---|
| **Toxicity** | **Ivaltinostat Dose Modification** | **Capecitabine Dose Modification** |
| Grade 1 | No dose adjustment | No dose adjustment |
| Grade 2 | Interrupt until Grade ≤2 | Interrupt until Grade ≤1 |
| | 1^{st} episode = RP2D mg/m² | 1^{st} episode = 800 mg/m² |
| | 2^{nd} episode = 80% of RP2D mg/m² | 2^{nd} episode = 600 mg/m² |
| | 3^{rd} episode = discontinue | 3^{rd} episode = discontinue |
| Grade 3 | Interrupt until Grade ≤2 | Interrupt until Grade ≤1 |
| | 1^{st} episode = 80% of RP2D mg/m² | 1^{st} episode = 800 mg/m² |
| | 2^{nd} episode = 64% of RP2D mg/m² | 2^{nd} episode = 600 mg/m² |
| | 3^{rd} episode = discontinue | 3^{rd} episode = discontinue |
| Grade 4 | Interrupt until Grade ≤2 | Interrupt until Grade ≤1 |
| | 1^{st} episode = 80% of RP2D mg/m² | |
| | | 1^{st} episode = 600 mg/m² |
| | 2^{nd} episode = discontinue | 2^{nd} episode = discontinue |

| | | |
|---|---|---|
| *Medical management should first be attempted. If nausea/vomiting persists despite optimal medical management, this table should be used to guide dose adjustments. | | |

**Table D**

| **LFT Abnormalities*** | | |
|---|---|---|
| **AST, ALT, Bilirubin Toxicities (NOT ALP)** | **Ivaltinostat Dose Modification** | **Capecitabine Dose Modification** |
| Grade 1 | No dose adjustment | No dose adjustment |
| Grade 2 | No dose adjustment | Interrupt until Grade ≤1 |
| | | If baseline Grade 1: No dose adjustment |
| | | If baseline Grade 0: |
| | | 1^{st} episode = 800 mg/m² |
| | | 2^{nd} episode = 60 mg/m² |
| | | 3^{rd} episode = discontinue |
| Grade 3 | Interrupt until Grade ≤2 | Interrupt until Grade ≤1 |
| | 1^{st} episode = 80% of RP2D mg/m² | 1^{st} episode = 800 mg/m² |
| | 2^{nd} episode = 64% of RP2D mg/m² | 2^{nd} episode = 600 mg/m² |
| | 3^{rd} episode = discontinue | 3^{rd} episode = discontinue |
| Grade 4 | Interrupt until Grade ≤2 | Interrupt until Grade ≤1 |
| | | 1^{st} episode = 800 mg/m² |
| | 1^{st} episode = 64% of RP2D mg/m² | 2^{nd} episode = 600 mg/m² |
| | 2^{nd} episode = discontinue | 3^{rd} episode = discontinue |

| | | |
|---|---|---|
| ALT=alanine aminotransferase; AST=aspartate aminotransferase; ALP=alkaline phosphatase; LFT=liver function test *Evaluate for anatomic causes of LFT abnormalities (e.g., biliary obstruction) and consider treatment of the underlying cause if determined. For LFT abnormalities persisting despite optimal medical and procedural management, and not attributable to causes other than study drugs, this table should be used to guide dose adjustments. | | |

**Table E**

| **Other Toxicities** | | |
|---|---|---|
| **Toxicity** | **Ivaltinostat Dose Modification** | **Capecitabine Dose Modification** |
| Grade 1 | No dose adjustment | No dose adjustment |
| Grade 2 | Interrupt until Grade ≤1 | Interrupt until Grade ≤1 |
| | 1^{st} episode = 80% of RP2D mg/m² | 1^{st} episode = 800 mg/m² |
| | 2^{nd} episode = 64% of RP2D mg/m² | 2^{nd} episode = 600 mg/m² |
| | 3^{rd} episode = discontinue | 3^{rd} episode = discontinue |
| Grade 3 | Interrupt until Grade ≤1 | Interrupt until Grade ≤1 |
| | 1^{st} episode = 80% of RP2D mg/m² | 1^{st} episode = 800 mg/m² |
| | 2^{nd} episode = 64% of RP2D mg/m² | 2^{nd} episode = 600 mg/m² |
| | 3^{rd} episode = discontinue | 3^{rd} episode = discontinue |
| Grade 4 | Interrupt until Grade ≤1 | Interrupt until Grade ≤1 |
| | 1^{st} episode = 64% of RP2D mg/m² | 1^{st} episode = 600 mg/m² |
| | 2^{nd} episode = discontinue | 2^{nd} episode = discontinue |

### Pharmacokinetics and Pharmacodynamics of Ivaltinostat Oral Therapy

A further embodiment of the present disclosure relates to a method for treating or ameliorating cancer in a subject in need thereof, comprising orally administering an effective amount of ivaltinostat to the subject, wherein the oral administration provides a plasma Cmax of ivaltinostat ranging from about 80 µg/L to about 1800 µg/L. In some embodiments, the effective amount of ivaltinostat is described herein in the method of treating cancer section. Further discussion on the PK/PD of ivaltinostat oral therapy are disclosed in detail in Example 3.

In some embodiments, the effective amount of ivaltinostat is about 125 mg, and the Cmax of ivaltinostat is about 90 µg/L to about 250 µg/L, for example, about 90, 95, 100, 105, 110, 115, 120, 125, 130, 135, 140, 145, 150, 155, 160, 165, 170, 175, 180, 185, 190, 195, 200, 205, 210, 215, 220, 225, 230, 235, 240, 245, or 250 µg/L, or a range defined by any two of the preceding values. In further embodiments, the Cmax of ivaltinostat is about 150 µg/L. In such embodiments, the AUCₗₐₛₜ or AUC_{inf} of ivaltinostat is from about 400 µg.h/L to about 1000 µg.h/L, for example, about 400, 410, 420, 430, 440, 450, 460, 470, 480, 490, 500, 510, 520, 530, 540, 550, 560, 570, 580, 590, 600, 610, 620, 630, 640, 650, 660, 670, 680, 690, 700, 710, 720, 730, 740, 750, 760, 770, 780, 790, 800, 810, 820, 830, 840, 850, 860, 870, 880, 890, 900, 910, 920, 930, 940, 950, 960, 970, 980, 990, or 1000 µg.h/L, or a range defined by any two of the preceding values. In further embodiments, the AUCₗₐₛₜ or AUC_{inf} of ivaltinostat is from about 500 µg.h/L to about 850 µg.h/L, about 550 µg.h/L to about 750 µg.h/L, about 640 µg.h/L, or about 650 µg.h/L.

In some other embodiments, the effective amount of ivaltinostat is about 375 mg, and the Cmax of ivaltinostat is about 500 µg/L to about 1500 µg/L, for example, about 500, 510, 520, 530, 540, 550, 560, 570, 580, 590, 600, 610, 620, 630, 640, 650, 660, 670, 680, 690, 700, 710, 720, 730, 740, 750, 760, 770, 780, 790, 800, 810, 820, 830, 840, 850, 860, 870, 880, 890, 900, 910, 920, 930, 940, 950, 960, 970, 980, 990, 1000, 1050, 1100, 1150, 1200, 1250, 1300, 1350, 1400, 1450, or 1500 µg/L, or a range defined by any two of the preceding values. In further embodiments, the Cmax of ivaltinostat is about 750 µg/L to about 1250 µg/L, or about 975 µg/L, or about 1000 µg/L. In further embodiments, the AUCₗₐₛₜ or AUC_{inf} of ivaltinostat is from about 1200 µg.h/L to about 6000 µg.h/L, for example, about 1200, 1300, 1400, 1500, 1600, 1700, 1800, 1900, 2000, 2100, 2200, 2300, 2400, 2500, 2600, 2700, 2800, 2900, 3000, 3100, 3200, 3300, 3400, 3500, 3600, 3700, 3800, 3900, 4000, 4100, 4200, 4300, 4400, 4500, 4600, 4700, 4800, 4900, 5000, 5100, 5200, 5300, 5400, 5500, 5600, 5700, 5800, 5900, or 6000 µg.h/L, or a range defined by any two of the preceding values. In further embodiments, the AUCₗₐₛₜ or AUC_{inf} of ivaltinostat is from about 2000 µg.h/L to about 4500 µg.h/L, from about 3000 µg.h/L to about 4000 µg.h/L, about 3700 µg.h/L or about 3740 µg.h/L.

In some other embodiments, the effective amount of ivaltinostat is about 750 mg, and the Cmax is about 750 µg/L to about 1800 µg/L, for example, about 750, 760, 770, 780, 790, 800, 810, 820, 830, 840, 850, 860, 870, 880, 890, 900, 910, 920, 930, 940, 950, 960, 970, 980, 990, 1000, 1050, 1100, 1150, 1200, 1250, 1300, 1350, 1400, 1450, 1500, 1550, 1600, 1650, 1700, 1750, or 1800 µg/L, or a range defined by any two of the preceding values. In further embodiments, the Cmax of ivaltinostat is from about 1000 µg/L to about 1500 µg/L, from about 1100 µg/L to about 1400 µg/L, or about 1225 µg/L. In further embodiments, the AUCₗₐₛₜ or AUC_{inf} of ivaltinostat is from about 4000 µg.h/L to about 10000 µg.h/L, for example, about 4000, 4500, 4600, 4700, 4800, 4900, 5000, 5100, 5200, 5300, 5400, 5500, 5600, 5700, 5800, 5900, 6000, 6100, 6200, 6300, 6400, 6500, 6600, 6700, 6800, 6900, 7000, 7100, 7200, 7300, 7400, 7500, 7600, 7700, 7800, 7900, 8000, 8100, 8200, 8300, 8400, 8500, 8600, 8700, 8800, 8900, 9000, 9100, 9200, 9300, 9400, 9500, 9600, 9700, 9800, 9900,or 10000 µg.h/L, or a range defined by any two of the preceding values. In further embodiments, the AUCₗₐₛₜ or AUC_{inf} of ivaltinostat is from about 5000 µg.h/L to about 8500 µg.h/L, from about 6000 µg.h/L to about 7500 µg.h/L, about 7100 µg.h/L, or about 7060 µg.h/L.

In some embodiments, the mean bioavailability of oral ivaltinostat is from about 7.5% to about 15%, for example, about 7.5, 8.0, 8.5, 9.0, 9.5, 10.0, 10.5, 11.0, 11.5, 12.0, 12.5, 13.0, 13.5, 14.0, 14.5, 15.0, or a range defined by any two of the preceding values. In further embodiments, the mean bioavailability of oral ivaltinostat is from about 7.7% to about 13.4%, about 10.9% or about 10.6%. In further embodiments, ivaltinostat is administered in a fast state.

In any embodiments of the method described herein, ivaltinostat is for administration as a combination therapy with one or more additional anti-cancer agents. In further embodiments, the ivaltinostat combination therapy is for use in providing maintenance treatment for pancreatic cancer (e.g., advanced or metastatic pancreatic adenocarcinoma). Ivaltinostat may be administered either by intravenous infusion (IV) or orally. In some embodiments, ivaltinostat is orally administered when a subject is in a fast state. In other embodiment, ivaltinostat is orally administered when a subject is in a fed state.

### Pharmaceutical Compositions

Ivaltinostat as described herein may be provided for administration in the form of a pharmaceutical composition, include an effective amount of ivaltinostat or a pharmaceutically acceptable salt, such as phosphate salt, and at least one pharmaceutically acceptable excipient or carrier.

The term "pharmaceutical composition" refers to a mixture of one or more compounds and/or salts disclosed herein with other chemical components, such as one or more excipients. The pharmaceutical composition facilitates administration of the compound to an organism. Pharmaceutical compositions will generally be tailored to the specific intended route of administration.

As used herein, an "excipient" refers to essentially inert substances that are added to a pharmaceutical composition to provide, without limitation, bulk, consistency, stability, binding ability, lubrication, disintegrating ability etc., to the composition. For example, stabilizers such as anti-oxidants and metal-chelating agents are excipients. Excipients also include ingredients in a pharmaceutical composition that lack appreciable pharmacological activity but may be pharmaceutically necessary or desirable. For example, to increase the bulk of a potent drug whose mass is too small for manufacture and/or administration. It may also be a liquid for the dissolution of a drug to be administered by injection, ingestion or inhalation. For example, a buffered aqueous solution such as, without limitation, phosphate buffered saline that mimics the pH and isotonicity of human blood.

The pharmaceutical compositions described herein can be administered to a human patient *per se,* or in pharmaceutical compositions where they are mixed with other active ingredients, as in combination therapy, or excipients, or combinations thereof. Proper formulation is dependent upon the route of administration chosen. Techniques for formulation and administration of the compounds described herein are known to those skilled in the art.

The pharmaceutical compositions disclosed herein may be manufactured in a manner that is itself known, *e.g.,* by means of conventional mixing, dissolving, granulating, dragee-making, levigating, emulsifying, encapsulating, entrapping or tableting processes. Additionally, the active ingredients (e.g., ivaltinostat and one or more additional anti-cancer agents) are contained in an amount effective to achieve its intended purpose.

In some embodiments, the pharmaceutical composition also contains at least one pharmaceutically acceptable inactive ingredient. The pharmaceutical composition can be formulated for intravenous injection, subcutaneous injection, oral administration, buccal administration, inhalation, nasal administration, topical administration, transdermal administration, ophthalmic administration, or otic administration. The pharmaceutical composition can be in the form of a tablet, a pill, a capsule, a liquid, an inhalant, a nasal spray solution, a suppository, a suspension, a gel, a colloid, a dispersion, a solution, an emulsion, an ointment, a lotion, an eye drop, or an ear drop.

Multiple techniques of administering a pharmaceutical composition exist in the art including, but not limited to, oral, rectal, pulmonary, topical, aerosol, injection, infusion and parenteral delivery, including intramuscular, subcutaneous, intravenous, intramedullary injections, intrathecal, direct intraventricular, intraperitoneal, intranasal and intraocular injections. In some embodiments, a pharmaceutical composition of ivaltinostat may be administered intravenously .In some other embodiments, a pharmaceutical composition of ivaltinostat may be administered orally.

The compositions may, if desired, be presented in a pack or dispenser device which may contain one or more unit dosage forms containing the active ingredient. The pack may for example comprise metal or plastic foil, such as a blister pack. The pack or dispenser device may be accompanied by instructions for administration. The pack or dispenser may also be accompanied with a notice associated with the container in form prescribed by a governmental agency regulating the manufacture, use, or sale of pharmaceuticals, which notice is reflective of approval by the agency of the form of the drug for human or veterinary administration. Such notice, for example, may be the labeling approved by the U.S. Food and Drug Administration for prescription drugs, or the approved product insert. Pharmaceutical Compositions as described herein formulated in a compatible pharmaceutical excipient may also be prepared, placed in an appropriate container, and labeled for treatment of an indicated condition. For ivaltinostat combination therapy, the pharmaceutical composition comprising ivaltinostat may be separate from a pharmaceutical composition containing the one or more additional therapeutic agents (e.g., one or more anti-cancer agents. For example, the pharmaceutical composition containing ivaltinostat is for administration by IV while the pharmaceutical composition containing the additional anti-cancer agent is for administration by a different route of administration (e.g., orally). In some such embodiments, the pharmaceutical composition of ivaltinostat may be separately packaged with the additional therapeutical agent(s) in a kit or marketed separately as two individual products. In other embodiments, the pharmaceutical composition containing ivaltinostat is administered by the same route as that of the pharmaceutical composition containing the additional anti-cancer agent(s). As such, ivaltinostat and the additional anti-cancer agent(s) may be in a single unit dosage form or may be adapted to be administered together.

### EXAMPLES

Additional embodiments are disclosed in further detail in the following examples, which are not in any way intended to limit the scope of the claims.

### Example 1. Phase 1b/2 Study of ivaltinostat in combination with capecitabine or capecitabine monotherapy

The primary objective of this Phase 1b/2 study is to determine the efficacy, as measured by PFS, of ivaltinostat in combination with capecitabine and capecitabine monotherapy in patients with metastatic pancreatic adenocarcinoma for maintenance treatment after disease response or stability with a FOLFIRINOX chemotherapy regimen.

In Phase 1b, 3 dose levels of ivaltinostat will be studied in combination with a fixed dose of capecitabine to determine the recommended Phase 2 dose (RP2D) of ivaltinostat.

In Phase 2, patients will be randomized in a 1:1 ratio to the combination of ivaltinostat and capecitabine or to capecitabine monotherapy. A fixed dose for capecitabine 1000 mg/m² orally twice daily will be taken on Days 1 to 14, and the RP2D of ivaltinostat will be administered intravenously once a week for 2 weeks, followed by 1 week of rest. One cycle consists of 21 days. Tumor response during study treatment will be assessed every 6 weeks up to Cycle10, then every 9 weeks afterwards using RECIST v1.1 criteria.

All eligible patients will have a histologically or cytologically confirmed diagnosis of metastatic pancreatic adenocarcinoma, must have received a minimum of 16 weeks of FOLFIRINOX treatment and must have no evidence of progression based on the Investigator's opinion. Screening will be completed no less than 2 and no more than 6 weeks after the last dose of first line chemotherapy. Randomization will occur within 6 weeks of the last dose of chemotherapy. Treatment will commence within 7 days of randomization.

There will be approximately 70 (18 Phase 1b dose-escalation, 52 Phase 2 randomized) patients in the study. In Phase 1b, 3 dose levels of ivaltinostat will be studied in combination with a fixed dose of capecitabine to determine the RP2D. In Phase 2, patients will be randomized in a 1:1 ratio to the combination of ivaltinostat and capecitabine or to capecitabine monotherapy as specified below:
Arm A
   - Ivaltinostat (the dose will be selected after reviewing the results of Phase 1b among 3 dose levels of 60, 125, or 250 mg/m²) intravenous infusion over 60 minutes (±10 minutes) once on Days 1 and 8 of a 21-day cycle
   - Capecitabine 1000 mg/m² orally twice daily on Days 1 to 14 of a 21-day cycle
      OR
Arm B
   - Capecitabine 1000 mg/m² orally twice daily on Days 1 to 14 of a 21-day cycle

In Phase 1b, patients attend clinic visits in Cycle 1 on Days 1, 2, 3, 5, 8, and 15 for assessments, with treatment on Days 1 and 8. After Cycle 1, patients attend clinic visits on a weekly schedule for treatment (Days 1 and 8) and assessments (Days 1, 8, and 15) during 21-day cycles for the remainder of time receiving study treatment. In Phase 2, patients attend clinic visits on a weekly schedule for treatment (Days 1 and 8) and assessments (Days 1, 8, and 15) during 21-day cycles.

The Phase 1b enroll up to 18 patients (6 patients each of 3 dose levels of ivaltinostat [60, 125, and 250 mg/m²] + 1000 mg/m² capecitabine BID) to assess the combination of ivaltinostat and capecitabine for safety and tolerability as well as to determine the ivaltinostat RP2D, assess the pharmacodynamics (PDy) of ivaltinostat, and assess PK parameters of both ivaltinostat and capecitabine. Data from the Phase 1b will be used in safety analyses but not in efficacy analyses.

Tumor response during study treatment will be assessed using RECIST v1.1 criteria. Baseline and on-treatment tumor assessments will be performed using CT or MRI scans with contrast of the chest, abdomen, and pelvis, with other regions as clinically indicated for the assessment of disease. Baseline evaluation should be performed within the 28-day screening period prior to the start of study treatment, as close as possible to randomization. Follow-up assessment consistent with baseline radiologic evaluation (i.e., if CT scan was performed for baseline assessment, then CT scan should be done for the follow-up evaluation) should be performed approximately every 6 weeks (±1 week) until objective disease progression as defined by RECIST v1.1. In addition to the imaging listed above, any other sites with known disease, or at which new disease is suspected, should also be appropriately imaged. Safety evaluations will occur at each protocol-specified study visit.

Patients will continue to receive study treatment until objective radiographic disease progression per RECIST v1.1 as assessed by the Investigator or until unacceptable toxicity occurs.

Once patients have discontinued study treatment, subsequent treatment options will be at the discretion of the treating physician. It is anticipated (but not required) that patients may be retreated with their first line regimen. Patients will be contacted on an approximately every-8-week schedule and followed up for survival. Details of any further anti-cancer treatment will be collected until death, loss to follow-up, or withdrawal of consent. In addition to contact every 8 weeks, patients will be contacted in the 7 days following a specified date (data cut-off date) to capture survival status at that point for each survival analysis. Any patient who discontinues study treatment for reasons other than objective radiographic progression should continue to undergo scheduled objective tumor assessments according to the study plan in order to assess objective radiographic progression of disease.

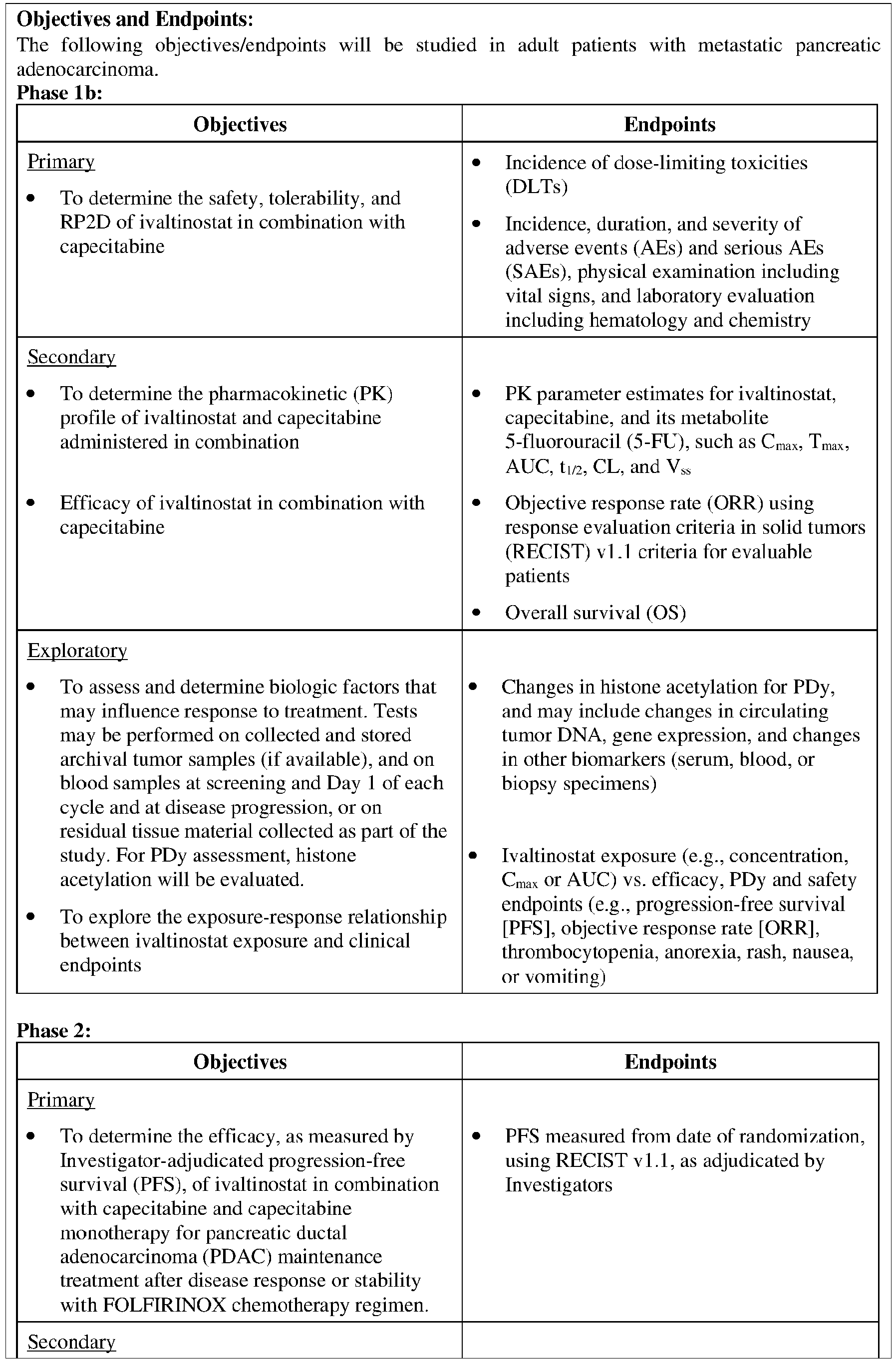

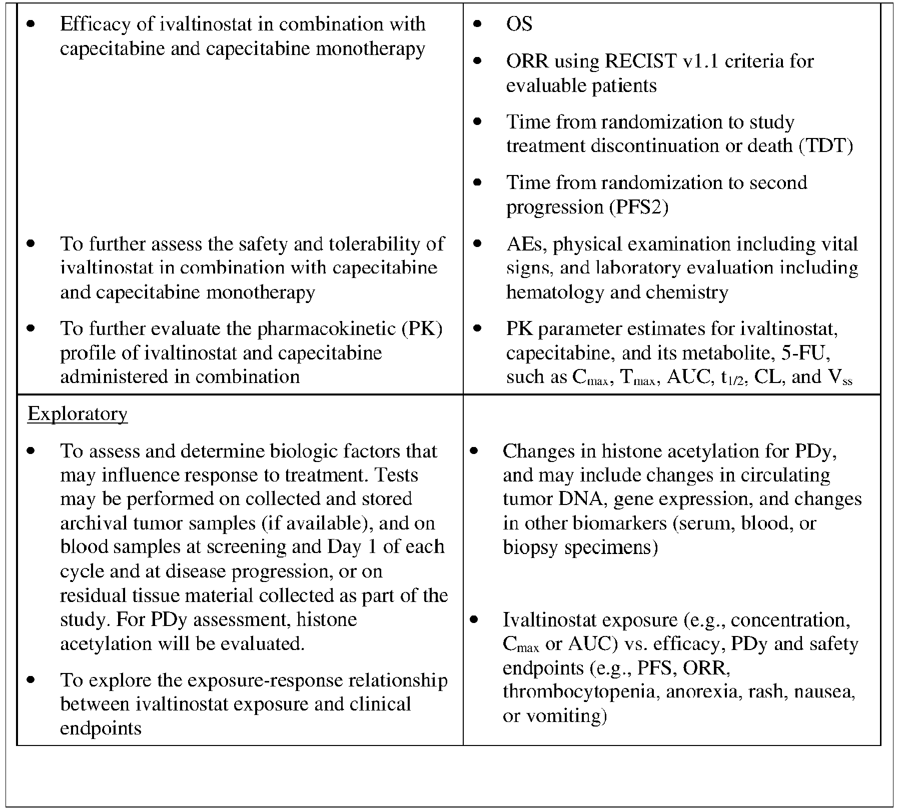

### Study Population

### Inclusion Criteria

1. Provision of informed consent prior to any study specific procedures.
2. Age: ≥18 years
3. For Phase 1b, histologically or cytologically confirmed pancreatic adenocarcinoma (locally advanced or metastatic) with at least 1 prior therapy in either the advanced or perioperative setting
4. For Phase 1b, measurable disease and/or non-measurable disease per RECIST v1.1
5. For Phase 2, histologically or cytologically confirmed pancreatic adenocarcinoma without evidence of disease progression while receiving initial chemotherapy for metastatic disease (e.g., must have had a demonstrated CR, PR, or SD following initial chemotherapy).
6. For Phase 2, measurable disease and/or non-measurable or no evidence of disease assessed by baseline CT (or MRI where CT is contraindicated). RECIST v1.1 will be used to allow for assessment of disease progression due to new lesions in patients with no evidence of disease at baseline. Patients with no evidence of disease following FOLFIRINOX chemotherapy will be deemed to have radiographic disease progression if new lesions are detected.
7. For Phase 2, treatment with FOLFIRINOX for metastatic pancreatic adenocarcinoma at full or modified doses, for a minimum of 16 weeks, and no evidence of progression based on the radiographic imaging.
   a. Randomization must occur within 6 weeks of the last dose of chemotherapy.
   b. Patients who have received at least 16 weeks of FOLFIRINOX combination regimen but had nonfluoropyrimidine chemotherapeutic agents discontinued prior to 16 weeks due to toxicity are eligible if they have no radiographic evidence of disease.
8. For Phase 2, patients who received prior chemotherapy or prior chemoradiation for a prior cancer or as adjuvant/neoadjuvant treatment for pancreatic adenocarcinoma are eligible provided at least 12 months have elapsed between the last dose of treatment and initiation of the FOLFIRINOX chemotherapy for metastatic pancreatic adenocarcinoma.
9. Prior radiation therapy is allowed, provided >14 days have elapsed since completion of radiation prior to randomization.
10. Normal organ function and bone marrow function as defined below:
   a. Hemoglobin ≥9.0 g/dL with no packed red blood cell transfusions in the past 7 days
   b. Absolute neutrophil count (ANC) ≥1.5 x 10⁹/L
   c. Platelet count ≥100 x 10⁹/L with no platelet transfusions in the past 7 days
   d. Total bilirubin ≤1.5 x institutional upper limit of normal (ULN)
   e. AST (SGOT)/ALT (SGPT) ≤2.5 x institutional ULN value unless liver metastases are present in which case it must be ≤5 x ULN
   f. Calculated creatinine clearance ≥50 mL/min by Cockroft-Gault formula. A 24-hour urine collection for creatinine clearance may also be used.
   g. Serum albumin ≥2.5 mg/dL
11. ECOG Performance Status 0-1 at the date of signing the informed consent.
12. Postmenopausal or evidence of non-childbearing status for women of childbearing potential: negative urine or serum pregnancy test. Postmenopausal is defined as:
   a. Amenorrheic for ≥1 year following cessation of exogenous hormonal treatments.
   b. Luteinizing hormone (LH) and follicle stimulating hormone (FSH) levels in the post-menopausal range for women under 50 years old.
   c. Radiation-induced ovarian ablation with last menses ≥1 year ago.
   d. Chemotherapy-induced menopause with ≥1-year interval since last menses.
   e. Surgical sterilization (bilateral oophorectomy or hysterectomy).
13. Women of childbearing potential who do not meet the above criteria must have no planned pregnancy in the next 6 months and be willing to take effective measures to prevent pregnancy with at least 2 forms of contraception. Options include: 1) hormonal method of birth control (insertable, injectable, transdermal, or combination oral [estrogen+ progestin]) + male condom; 2) intrauterine contraceptive device + male condom; 3) tubal occlusion + male condom; 4) vasectomized sexual partner (with participant assurance that partner received post-vasectomy confirmation of azoospermia) + male condom; and 5) true total sexual abstinence from the first dose of study drug to 90 days after the last dose.
14. Male patients must agree to abstain from sexual intercourse or to use double-barrier contraception (as above) for the duration of the treatment period in the study and for at least 90 days after receiving the last dose of study medication. Male patients should also avoid semen donation or providing semen for in-vitro fertilization during the above-mentioned duration.
15. Willing to comply with the protocol for the duration of the study including undergoing treatment and scheduled visits and examinations.

### Exclusion Criteria

1. For Phase 2, radiographic progression of tumor per RECIST 1.1 between start of first line FOLFIRINOX chemotherapy for metastatic pancreatic adenocarcinoma and randomization.
2. Cytotoxic chemotherapy or non-hormonal targeted therapy within 28 days of Cycle 1 Day 1 is not permitted. Palliative radiotherapy must have been completed 14 or more days before Cycle 1 Day 1. The patient can receive a stable dose of bisphosphonates or RANKL-directed therapy for bone metastases before and during the study as long as these were initiated at least 2 weeks prior to study treatment.
3. For Phase 2, not receiving FOLFIRINOX as initial therapy for metastatic PDAC. Patients who received FOLFIRINOX initially and who needed to discontinue irinotecan or oxaliplatin due to toxicity are eligible, provided they received at least 4 weeks (2 cycles) of FOLFIRINOX.
4. For Phase 2, more than 1 prior line of therapy for metastatic PDAC.
5. Exposure to an investigational agent within 30 days or 5 half-lives (whichever is longer) prior to randomization.
6. Any previous treatment with a histone deacetylase (HDAC) inhibitor, including ivaltinostat.
7. Other primary cancers. EXCEPTIONS: adequately treated non-melanoma skin cancer, prostate cancer s/p resection with an undetectable prostate-specific antigen (PSA), curative treated in-situ cancer of the cervix, ductal carcinoma in situ (DCIS) of the breast, Stage 1 Grade 1 endometrial carcinoma, or other solid tumors including lymphomas (without bone marrow involvement) curatively treated with no evidence of disease for ≥2 years prior to study entry.
8. Clinically significant (in the judgement of the Investigator) persistent toxicities (CTCAE v5.0 Grade ≥2) caused by previous cancer therapy, excluding alopecia and Grade 2 neuropathy.
9. Major surgery within 2 weeks of starting treatment. Patients must also have recovered from effects of any major surgery.
10. Patients with average QTc (using Fridericia correction calculation) >470 msec
11. Known active hepatitis B or C infection (positive HBV surface antigen, positive HCV RNA). Patients with treated hepatitis B or C and no evidence of active viral replication may participate.
12. Known HIV infection.
13. Clinically significant uncontrolled medical conditions are not permitted (e.g., active infection requiring IV antibiotics), symptomatic congestive heart failure (NYHA Class III or IV;), unstable angina pectoris, recent (3 months) myocardial infarction, extensive bilateral interstitial lung disease, psychiatric illness that would limit ability to comply with study procedures, and any other medical condition that, in the opinion of the Investigator, places the patient at unacceptable risk of toxicity. Patients with venous thromboembolism (VTE) (pulmonary embolism [PE] or deep vein thrombosis [DVT]) are allowed to participate if they have been on stable anticoagulation for at least 3 months.
14. Refractory ascites (requiring regular paracenteses at intervals <2 weeks).
15. Gastrointestinal hemorrhage requiring blood transfusion within 30 days prior to randomization.
16. Known central nervous system (CNS) metastases or leptomeningeal disease. Patients who have had adequately treated CNS metastases and are no longer on steroids or anticonvulsant therapy are eligible.
17. Patient unable to swallow orally administered medication and/or gastrointestinal disorders likely to interfere with absorption of the study medication.
18. Pregnant or breast-feeding women.
19. Known hypersensitivity to ivaltinostat or capecitabine or any of the excipients of the agents.
20. Known dihydropyrimidine dehydrogenase (DPD) deficiency
21. Known pathogenic or likely pathogenic germline mutation in BRCA1 or BRCA2 genes.
22. Patients using strong inhibitors of CYP3A4 and CYP2D6 or drugs recommended to not be co-administered with capecitabine.
23. Patients using inhibitors of cytidine deaminase.
24. Patients using full dose coumadin/warfarin for anticoagulation.

### Study Drug/Treatment Groups, Dose, and Mode of Administration

Ivaltinostat will be supplied as 125 mg powder in glass vials and reconstituted with 0.9% normal saline. Ivaltinostat 60, 125, or 250 mg/m² will be IV infused over 60 minutes (±10 minutes) on Days 1 and 8 of a 21-day cycle (Phase 1b and Phase 2 Arm A).

Capecitabine is commercially available in 150 and 500 mg tablets. Capecitabine 1000 mg/m2 will be administered orally twice daily on Days 1 to 14 of a 21-day cycle. Capecitabine should be administered within 30 minutes after a meal and, on days of ivaltinostat infusions, within 10 minutes prior to the start of the ivaltinostat IV infusion.

### Duration of Treatment/Study

Each patient will continue study treatment until progressive disease (PD), toxicity, death, or discontinuation based on the patient's or Investigator's decision.

Total duration is up to approximately 34 months, which includes a screening period of 28 days, a treatment period of approximately 8 months (approximately 10 cycles of treatment), a safety follow-up period of approximately 30 days, followed by a post study Long-Term Survival follow-up for up to approximately 2 years (approximately every 8 weeks after the safety follow-up visit).

### Dose Modifications

In the event of an AE at least possibly related to either of the study drugs, the doses of ivaltinostat and/or capecitabine should be adjusted according to the guidelines shown in the following tables. If an AE at least possibly related is not covered in the table, doses may be reduced or held at the discretion of the Investigator for the patient's safety. Of note, patients who experience a recurrent treatment-related AE requiring dose reduction of either/both agents more than 2 times should be taken off study.

As the 2 study drugs do exhibit potentially overlapping toxicities, in situations where drug-specific attribution of an AE is not clear and the order of dose-reduction rules is not otherwise specified, both drugs may be reduced simultaneously at the discretion of the Investigator; or each drug may be reduced in turn in alternating fashion (i.e., first try a dose reduction of ivaltinostat; then a dose reduction in capecitabine; etc.).

If one study drug is held or discontinued due to AE, the other medication may be continued at the discretion of the treating physician after discussion with the study Medical Monitor. Once a dose reduction of a drug has occurred, it will not be re-escalated to its starting dose or prior dose level.

Patients with AEs that are manageable with supportive therapy may not require dose reductions (e.g., nausea/vomiting may be treated with antiemetics, diarrhea may be treated with loperamide, anemia may be managed with transfusions, electrolyte abnormalities may be corrected with supplements rather than by dose reduction).

### Dose Levels (Each drug is considered separately)

| **Dose Level** | **Ivaltinostat** | **Capecitabine** |
|---|---|---|
| 0 | RP2D mg/m² | 1000 mg/m² |
| -1 | 80% of RP2D mg/m² | 800 mg/m² |
| -2 | 64% of RP2D mg/m² | 600 mg/m² |

### Example 2. Phase 1/2 Study of ivaltinostat (IV) in combination with gemcitabine and erlotinib (illustrative)

In the Phase 1 component of the study, a total of 10 subjects received ivaltinostat at 3 different dose levels: 187.5 mg/m² (n=3), 250 mg/m² (n=3), and 312.5 mg/m² (n=4). Ivaltinostat was administered on Days 1, 8 and 15 of a 28-day cycle in combination with gemcitabine (1000 mg/m²; IV on Days 1, 8, and 15) and erlotinib (100 mg orally QD). All 10 subjects completed cycle 1 and were eligible for dose limiting toxicity (DLT) evaluation.

In the Phase 2 component of the study, 24 subjects received ivaltinostat at a dose level of 250 mg/m² in combination of gemcitabine (1000 mg/m²; IV on Days 1, 8, and 15) and erlotinib (100 mg orally QD). In Phase 2, planned treatment duration was up to 6 cycles, but treatment could continue beyond 6 cycles if there was no progression of disease, at the discretion of the subject and treating physician. Ten subjects completed the study goal of 6 cycles of treatment; four subjects continued treatment beyond 6 cycles.

In the Phase 2 portion of the study, ivaltinostat 250 mg/m² plus gemcitabine and erlotinib was found to be generally well tolerated, with myelosuppression as the primary toxicity. The most common grade ≥3 treatment-related AEs (frequency ≥10%) were neutrophil count decreased (13% grade 3; 17% grade 4), platelet count decreased (17% grade 3; 13% grade 4), and anemia (17% grade 3; 0% grade 4). No subjects discontinued study drug due to an AE and there were no deaths reported related to SAEs that were considered related to the investigational drugs. Among the 16 subjects who had at least one tumor evaluation, the objective response rate (ORR) was 25.0% and the disease control rate (DCR) was 93.8%, based on partial responses in 4 patients (25%) and stable disease in 11 patients (68.8%).

### Example 3. A Random Phase 1, Single-Dose Study of Ivaltinostat (IV and Oral)

The objective of this study was to evaluate the pharmacokinetics (PK), pharmacodynamics (PD) and tolerability of IV (CG-745) and oral (CG-750) formulations of Ivaltinostat in healthy volunteers.

### Study Design

A randomized double-blind, placebo-controlled, two-treatment, two-period crossover study was conducted in 3 cohorts. Subjects were randomized (6:2) to receive either CG-745 (cohort 1 and 3: 125 mg; cohort 2: 250 mg) or placebo, followed by CG-750 (cohort 1: 125 mg; cohort 2: 375 mg; cohort 3: 750 mg) or placebo after a 14-day washout period Additionally, food effect was investigated in cohort 2 in the same design. FIG. 1 illustrates the study design in detail. Healthy male volunteers aged 19-50 years with body mass index of 18-30 kg/m2 were eligible for the study.

### Pharmacokinetic Sampling, Assay and Analysis

CG-745 (IV): 0 (pre-dose), 0.33 (20 min), 0.67 (40 min), 1, 1.083 (1 h 5 min), 1.25 (1 h 15 min), 1.5, 2, 3, 4, 5, 6, 7, 9, 12, 25, 48, and 72 hours post beginning of IV infusion.

CG-750 (PO): 0 (pre-dose), 0.33 (20 min), 0.67 (40 min), 1, 1.5, 2, 3, 4, 5, 6, 7, 9, 12, 25, 48, and 72 hours post oral administration.

Urine samples for ivaltinostat were collected at the time interval of 0-4, 4-12, 12-24-, and 24-48-hours post-dose during each period. Ivaltinostat was quantified with validated liquid chromatography-tandem mass spectrometry (LC/MS/MS).

PK parameters including the maximum plasma concentration (Cₘₐₓ) and the area under the concentration-time curve from time 0 to time of the last quantifiable concentration (AUCₗₐₛₜ) were calculated by noncompartmental method. Bioavailability (F) was calculated by the ratio (PO/IV) of dose-normalized AUC_{inf} within each cohort. Dose-normalized parameters (Cₘₐₓ/Dose, AUCₗₐₛₜ/Dose) from each cohort were analyzed by Kruskal-Wallis test, and dose proportionality was assessed with a power model (Ln(y)=α + ß*Ln[Dose]).

### Pharmacodynamic Sampling and Analysis

Histone H3 acetylation at sites lysine 9 (K9), lysine 9/14 (K9/K14), and lysine 27 (K27) from peripheral blood mononuclear cells (PBMCs) were evaluated by western blot, Blood samples were collected for H3 acetylation at 0 (pre-dose), 2, 5, 9, 25, 48, and 72 hours post administration. AUEC, area under the %induction of histone acetylation vs time curve, was calculated by noncompartmental analysis using PhoenixTM WinNonlin^{®} (Pharsight, CA, USA) software version 8.0. Pharmacodynamic effects of ivaltinostat on AUEC was compared between the two formulations (CG-745 vs CG-750 in fasted state) within each cohort using Wilcoxon signed-rank test.

### Safety and Tolerability

The safety and tolerability were assessed through vital signs, physical examinations, clinical laboratory tests, 12-lead electrocardiogram (ECG) and adverse event (AE) monitoring.

### Study Results

A total of 25 subjects were randomized and 23 completed the study. One patient in cohort 2 did not receive treatment due to consent withdrawal, and one subject in placebo group (cohort 2) withdrew consent after period 1 and 2 treatments.

### Pharmacokinetic Results

The mean bioavailability of CG-750 in fasted state was 7.68%, 13.36%, and 10.86% for cohort 1, 2 and 3, respectively. The elimination half-life of ivaltinostat after a single administration of CG-745 (IV) was consistent between cohorts, ranging from 13.36 to 14.82h. After administration of CG-750 (PO), the elimination half-life ranged from 8.77 to 14.41h. The difference of Cₘₐₓ between cohorts was insignificant (p = 0.0516), and the slope of regression line (95% CI) analyzed by power model showed 1.2199 (0.8477 ~ 1.5920). The AUCₗₐₛₜ and AUC_{inf} for CG-750 did not meet the criteria for dose-proportionality but showed dose-linearity [p = 0.0691 for both AUCₗₐₛₜ and AUC_{inf}; slope of regression line (95% CI) was 1.3692 (1.0486 ~ 1.6898) and 1.3643 (1.0457 - 1.6830) for AUCₗₐₛₜ and AUC_{inf} respectively. Detailed PK data are summarized in Table 1 below.

### Pharmacokinetic Results

Immediately after CG-745 (IV) or CG-750 (PO) administration, a marked induction in histone H3 acetylation was observed at all studied cohorts. AUECs (K9, K9/K14 or K27) were ranged 4,422 to 18,749 %. h and 321 to 13,714 %·h after the administration of CG-745 (IV) and CG-750 (PO) in fasted state, respectively. Ivaltinostat AUCₗₐₛₜ and histone acetylation AUEC was generally well correlated. AUEC of all acetylation sites in cohort 3 were comparable between CG-745 IV and CG-750 (*p* = 0.3125, 0.8438, 0.3125 for K9, K9/14, K27, respectively), with CG-750 showing comparable or higher acetylation in all three comparisons as summarized in Table 2 below.

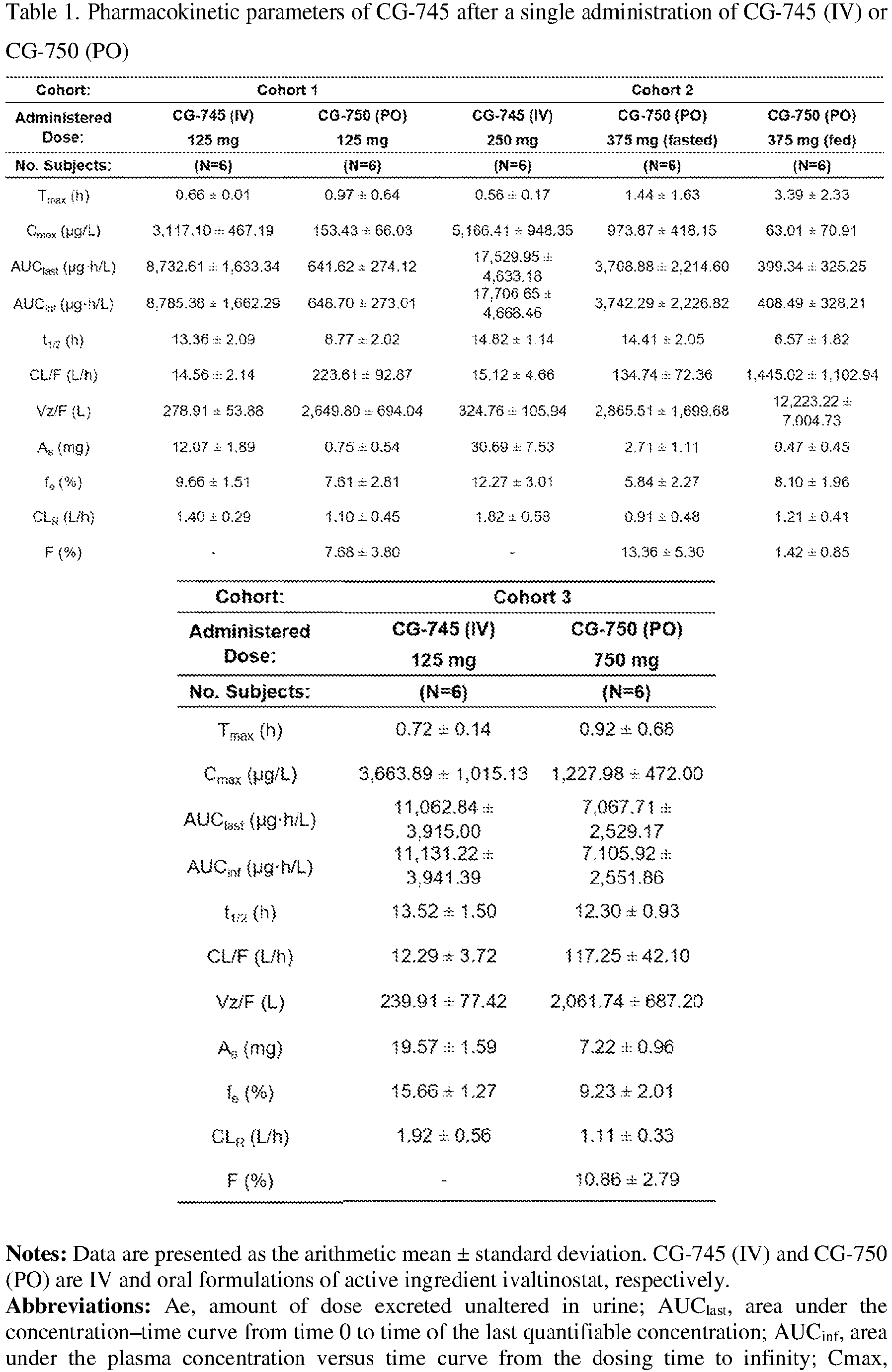

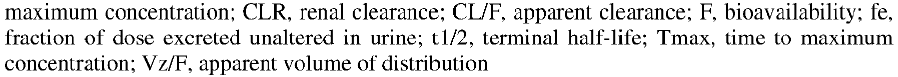

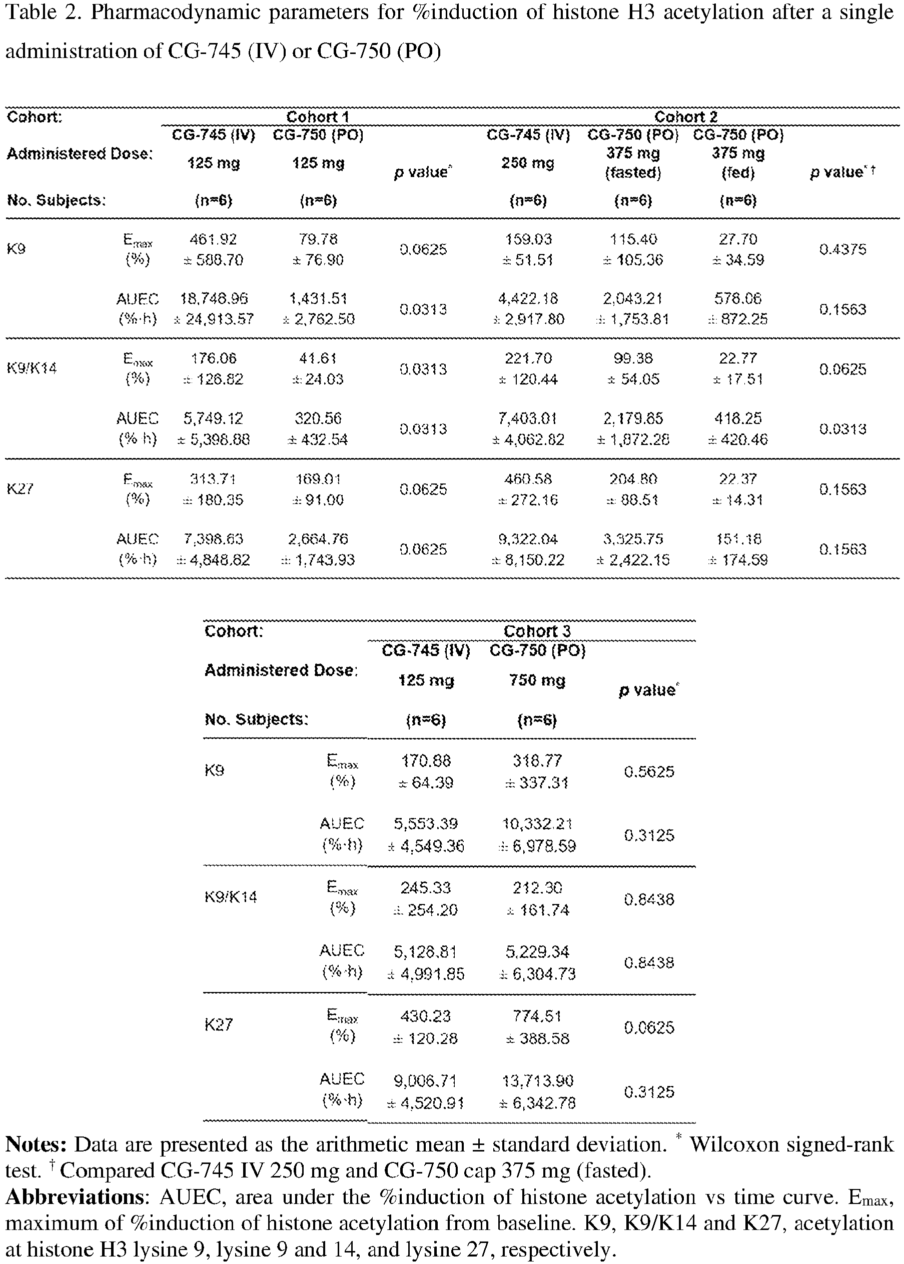

### Safety and Tolerability Results

There were no serious AEs reported and no clinically significant findings were observed in vital signs, physical examinations, clinical laboratory tests and ECGs. Most common adverse events were leukopenia (23 events in 16 subjects) and neutropenia (23 events in 15 subjects). All AEs were consistent with those expected with the mechanism of the study drug, and all were resolved spontaneously without any sequelae.

### Conclusion

CG-750 oral formulation and CG-745 IV were generally well tolerated after a single oral and IV administration. The mean bioavailability of CG-750 was 10.6 % and displayed dose-dependent PKs and PDs.

## Claims

1. Ivaltinostat for use in a method of treating pancreatic cancer in a subject in need thereof, wherein the subject has been treated with a first line fluoropyrimidine-based chemotherapy, wherein ivaltinostat is for use in combination with one or more additional anti-cancer agents, wherein the one or more additional anticancer agents is capecitabine.

2. Ivaltinostat for use according to claim 1, wherein the subject's pancreatic cancer has a response or stability on a first line fluoropyrimidine-based chemotherapy, or the subject's pancreatic cancer has not progressed on a first line fluoropyrimidine-based chemotherapy.

3. Ivaltinostat for use according to claim 1 or 2, wherein ivaltinostat for use is in an amount from about 25 mg/m² to about 500 mg/m², from about 50 mg/m² to about 375 mg/m², about 60 mg/m², about 125 mg/m², or about 250 mg/m², or
in an amount from about 10 mg/day to about 1000 mg/day, about 50 mg/day, about 100 mg/day, about 200 mg/day, or about 300 mg/day.

4. Ivaltinostat for use according to any one of claims 1 to 3, wherein
- ivaltinostat is for administration by intravenous infusion, and wherein the intravenous infusion is over about 30 minutes to about 120 minutes, from about 50 minutes to about 70 minutes, or about 60 minutes; or
- ivaltinostat is for oral administration, and wherein ivaltinostat is for administration on day 1 and day 8 of a 21-day cycle.

5. Ivaltinostat for use according to any one of claims 1 to 4, wherein capecitabine for administration is in an amount from about 200 mg/m² to about 2000 mg/m², from about 500 mg/m² to about 1500 mg/m², or from about 750 mg/m² to about 1250 mg/m², or
wherein capecitabine for administration is in an amount of about 1000 mg/m².

6. Ivaltinostat for use according to claim 5, wherein capecitabine is for oral administration.

7. Ivaltinostat for use according to claim 5 or 6, wherein capecitabine is for administration twice daily.

8. Ivaltinostat for use according to any one of claims 1 to 7, wherein the pancreatic cancer is pancreatic adenocarcinoma, locally advanced pancreatic adenocarcinoma, or metastatic pancreatic adenocarcinoma.

9. Ivaltinostat for use according to any one of claims 1 to 8, wherein the subject has been treated with a FOLFIRINOX chemotherapy regimen.

10. Ivaltinostat for use according to claim 8 or 9, wherein the subject has metastatic pancreatic adenocarcinoma, and the subject has a response or stability with the first line fluoropyrimidine-based chemotherapy, or the subject has metastatic pancreatic adenocarcinoma whose disease has not progressed on a first line fluoropyrimidine based chemotherapy.

11. Ivaltinostat for use according to any one of claims 1 to 10, wherein the administration of ivaltinostat is discontinued if the subject's absolute neutrophil count (ANC) is less than about 1,000/µL or the platelet count is less than about 100,000/µL.

12. Ivaltinostat for use according to any one of claims 1 to 11, wherein if the subject suffers from one or more hematological adverse events related to ivaltinostat administration, the amount of ivaltinostat is reduced after the subject recovers from the one or more hematologic adverse events.

13. Ivaltinostat for use according to claim 12, wherein the hematologic adverse events comprise Grade 3 neutropenia or Grade 3 thrombocytopenia, and wherein the amount of ivaltinostat is reduced to about 80% when a second episode of Grade 3 neutropenia or Grade 3 thrombocytopenia occurs, or wherein ivaltinostat is discontinued when a third episode of Grade 3 neutropenia or Grade 3 thrombocytopenia occurs.

14. Ivaltinostat for use according to claim 12, wherein the hematologic adverse events comprise Grade 4 neutropenia, Grade 4 thrombocytopenia, or Grade 3 febrile neutropenia and
wherein the amount of ivaltinostat is reduced to about 80% when a first episode of Grade 4 neutropenia, Grade 4 thrombocytopenia, or Grade 3 febrile neutropenia occurs, or
wherein the amount of ivaltinostat is reduced to about 64% when a second episode of Grade 4 neutropenia, Grade 4 thrombocytopenia, or Grade 3 febrile neutropenia occurs, or
wherein ivaltinostat is discontinued when a third episode of Grade 4 neutropenia, Grade 4 thrombocytopenia, or Grade 3 febrile neutropenia occurs.

15. Ivaltinostat for use according to claim 12, wherein the hematologic adverse events comprise Grade 4 febrile neutropenia, and
wherein the amount of ivaltinostat is reduced to about 80% when a first episode of Grade 4 febrile neutropenia occurs, or
wherein ivaltinostat is discontinued when a second episode of Grade 4 febrile neutropenia occurs.

## Patentansprüche

1. Ivaltinostat zur Verwendung in einem Verfahren zur Behandlung von Bauchspeicheldrüsenkrebs bei einem Patienten, der dieses benötigt, wobei der Patient mit einer Fluorpyrimidin-basierten Erstlinien-Chemotherapie behandelt wurde, wobei Ivaltinostat in Kombination mit einem oder mehreren weiteren Antikrebsmitteln verwendet wird, wobei es sich bei dem einen oder den mehreren zusätzlichen Antikrebsmittel um Capecitabin handelt.

2. Ivaltinostat zur Verwendung gemäß Anspruch 1, wobei der Bauchspeicheldrüsenkrebs des Patienten auf die Fluorpyrimidin-basierte Erstlinien-Chemotherapie anspricht oder stabil bleibt, oder der Bauchspeicheldrüsenkrebs des Patienten bei der Fluorpyrimidin-basierten Erstlinien-Chemotherapie nicht fortgeschritten ist.

3. Ivaltinostat zur Verwendung gemäß Anspruch 1 oder 2, wobei Ivaltinostat zur Verwendung in einer Menge von etwa 25 mg/m² bis etwa 500 mg/m², von etwa 50 mg/m² bis etwa 375 mg/m², etwa 60 mg/m², etwa 125 mg/m², oder etwa 250 mg/m² oder in einer Menge von etwa 10 mg/Tag bis etwa 1000 mg/Tag, etwa 50 mg/Tag, etwa 100 mg/Tag, etwa 200 mg/Tag oder etwa 300 mg/Tag verwendet wird.

4. Ivaltinostat zur Verwendung gemäß einem der Ansprüche 1 bis 3, wobei
- Ivaltinostat für die Verabreichung mittels intravenöser Infusion bestimmt ist, und wobei die intravenöse Infusion über etwa 30 Minuten bis etwa 120 Minuten, von etwa 50 Minuten bis etwa 70 Minuten, oder etwa 60 Minuten erfolgt; oder
- Ivaltinostat für die orale Verabreichung bestimmt ist, und wobei Ivaltinostat für die Verabreichung an Tag 1 und Tag 8 eines 21-Tage-Zyklus bestimmt ist.

5. Ivaltinostat zur Verwendung gemäß einem der Ansprüche 1 bis 4, wobei Capecitabin in einer Menge von etwa 200 mg/m² bis etwa 2000 mg/m², von etwa 500 mg/m² bis etwa 1500 mg/m² oder von etwa 750 mg/m² bis etwa 1250 mg/m² verabreicht wird, oder
wobei Capecitabin in einer Menge von etwa 1000 mg/m² verabreicht wird.

6. Ivaltinostat zur Verwendung gemäß Anspruch 5, wobei Capecitabin zur oralen Verabreichung bestimmt ist.

7. Ivaltinostat zur Verwendung gemäß Anspruch 5 oder 6, wobei Capecitabin zweimal täglich verabreicht wird.

8. Ivaltinostat zur Verwendung gemäß einem der Ansprüche 1 bis 7, wobei der Bauchspeicheldrüsenkrebs ein Bauchspeicheldrüsen-Adenokarzinom, ein lokal fortgeschrittenes Bauchspeicheldrüsen-Adenokarzinom oder ein metastasiertes Bauchspeicheldrüsen-Adenokarzinom ist.

9. Ivaltinostat zur Verwendung gemäß einem der Ansprüche 1 bis 8, wobei der Patient mit einem FOLFIRINOX-Chemotherapie-Schema behandelt wurde.

10. Ivaltinostat zur Verwendung gemäß Anspruch 8 oder 9, wobei der Patient ein metastasiertes Bauchspeicheldrüsen-Adenokarzinom hat, und der Patient auf die Fluorpyrimidin-basierte Erstlinien-Chemotherapie anspricht oder stabil bleibt, oder der Patient ein metastasiertes Bauchspeicheldrüsen-Adenokarzinom hat, wobei die Erkrankung unter einer Fluorpyrimidin-basierte Erstlinien-Chemotherapie nicht fortgeschritten ist.

11. Ivaltinostat zur Verwendung gemäß einem der Ansprüche 1 bis 10, wobei die Verabreichung von Ivaltinostat nicht fortgesetzt wird, wenn die absolute Neutrophilenzahl (ANC) des Patienten weniger als etwa 1.000/µL beträgt oder die Thrombozytenzahl weniger als etwa 100.000/µL beträgt.

12. Ivaltinostat zur Verwendung gemäß einem der Ansprüche 1 bis 11, wobei, wenn der Patient an einem oder mehreren hämatologischen Nebenwirkungen in Zusammenhang mit der Ivaltinostat-Verabreichung leiden, die Menge an Ivaltinostat reduziert wird, nachdem sich der Patient von der einen oder den mehreren hämatologischen Nebenwirkungen erholt hat.

13. Ivaltinostat zur Verwendung gemäß Anspruch 12, wobei die hämatologischen Nebenwirkungen eine Neutropenie Grad 3 oder eine Thrombozytopenie Grad 3 umfassen, und wobei die Menge an Ivaltinostat auf etwa 80% reduziert wird, wenn eine zweite Episode einer Neutropenie Grad 3 oder einer Thrombozytopenie Grad 3 auftritt, oder wobei Ivaltinostat abgebrochen wird, wenn eine dritte Episode einer Neutropenie Grad 3 oder einer Thrombozytopenie Grad 3 auftritt.

14. Ivaltinostat zur Verwendung gemäß Anspruch 12, wobei die hämatologischen Nebenwirkungen eine Neutropenie Grad 4, eine Thrombozytopenie Grad 4 oder eine febrile Neutropenie Grad 3 umfassen und
wobei die Menge an Ivaltinostat auf etwa 80% reduziert wird, wenn eine erste Episode einer Neutropenie Grad 4, einer Thrombozytopenie Grad 4 oder einer febrilen Neutropenie Grad 3 auftritt, oder
wobei die Menge an Ivaltinostat auf etwa 64% reduziert wird, wenn eine zweite Episode einer Neutropenie Grad 4, einer Thrombozytopenie Grad 4 oder einer febrilen Neutropenie Grad 3 auftritt, oder
wobei Ivaltinostat abgebrochen wird, wenn eine dritte Episode einer Neutropenie Grad 4, einer Thrombozytopenie Grad 4 oder einer febrilen Neutropenie Grad 3 auftritt.

15. Ivaltinostat zur Verwendung gemäß Anspruch 12, wobei die hämatologischen Nebenwirkungen eine febrile Neutropenie Grad 4 umfassen, und
wobei die Menge an Ivaltinostat auf etwa 80% reduziert wird, wenn eine erste Episode einer febrilen Neutropenie Grad 4 auftritt, oder
wobei Ivaltinostat abgebrochen wird, wenn eine zweite Episode einer febrilen Neutropenie Grad 4 auftritt.

## Revendications

1. Ivaltinostat destiné à être utilisé dans un procédé de traitement du cancer du pancréas chez un sujet qui en a besoin, sachant que le sujet a été traité par une chimiothérapie de première ligne à base de fluoropyrimidine, sachant que l'ivaltinostat est destiné à être utilisé en combinaison avec un ou plusieurs agents anticancéreux supplémentaires, sachant que l'un ou les plusieurs agents anticancéreux supplémentaires est/sont la capécitabine.

2. Ivaltinostat destiné à être utilisé selon la revendication 1, sachant que le cancer du pancréas du sujet présente une réponse ou une stabilité suite à une chimiothérapie de première ligne à base de fluoropyrimidine, ou le cancer du pancréas du sujet n'a pas progressé suite à une chimiothérapie de première ligne à base de fluoropyrimidine.

3. Ivaltinostat destiné à être utilisé selon la revendication 1 ou 2, sachant que l'ivaltinostat destiné à être utilisé est dans une quantité d'environ 25 mg/m² à environ 500 mg/m², d'environ 50 mg/m² à environ 375 mg/m², d'environ 60 mg/m², d'environ 125 mg/m², ou d'environ 250 mg/m², ou dans une quantité d'environ 10 mg/jour à environ 1000 mg/jour, d'environ 50 mg/jour, d'environ 100 mg/jour, d'environ 200 mg/jour, ou d'environ 300 mg/jour.

4. Ivaltinostat destiné à être utilisé selon l'une quelconque des revendications 1 à 3, sachant que
- l'ivaltinostat est destiné à être administré par perfusion intraveineuse, et sachant que la perfusion intraveineuse dure environ 30 minutes à environ 120 minutes, d'environ 50 minutes à environ 70 minutes, ou environ 60 minutes ; ou
- l'ivaltinostat est destiné à être administré par voie orale, et sachant que l'ivaltinostat est destiné à être administré au jour 1 et au jour 8 d'un cycle de 21 jours.

5. Ivaltinostat destiné à être utilisé selon l'une quelconque des revendications 1 à 4, sachant que la capécitabine destinée à être administrée est dans une quantité d'environ 200 mg/m² à environ 2000 mg/m², d'environ 500 mg/m² à environ 1500 mg/m², ou d'environ 750 mg/m² à environ 1250 mg/m², ou
sachant que la capécitabine destinée à être administrée est dans une quantité d'environ 1000 mg/m².

6. Ivaltinostat destiné à être utilisé selon la revendication 5, sachant que la capécitabine est destinée à être administrée par voie orale.

7. Ivaltinostat destiné à être utilisé selon la revendication 5 ou 6, sachant que la capécitabine est destinée à être administrée deux fois par jour.

8. Ivaltinostat destiné à être utilisé selon l'une quelconque des revendications 1 à 7, sachant que le cancer du pancréas est un adénocarcinome pancréatique, un adénocarcinome pancréatique localement avancé, ou un adénocarcinome pancréatique métastatique.

9. Ivaltinostat destiné à être utilisé selon l'une quelconque des revendications 1 à 8, sachant que le sujet a été traité par un régime de chimiothérapie au FOLFIRINOX.

10. Ivaltinostat destiné à être utilisé selon la revendication 8 ou 9, sachant que le sujet présente un adénocarcinome pancréatique métastatique, et le sujet présente une réponse ou une stabilité avec la chimiothérapie de première ligne à base de fluoropyrimidine, ou le sujet présente un adénocarcinome pancréatique métastatique dont la maladie n'a pas progressé suite à une chimiothérapie de première ligne à base de fluoropyrimidine.

11. Ivaltinostat destiné à être utilisé selon l'une quelconque des revendications 1 à 10, sachant que l'administration d'ivaltinostat est interrompue si la numération absolue des neutrophiles (ANC) du sujet est inférieure à environ 1 000/µL ou si la numération plaquettaire est inférieure à environ 100 000/µL.

12. Ivaltinostat destiné à être utilisé selon l'une quelconque des revendications 1 à 11, sachant que, si le sujet souffre d'un ou de plusieurs événements indésirables hématologiques liés à l'administration d'ivaltinostat, la quantité d'ivaltinostat est réduite après que le sujet s'est rétabli de l'un ou des plusieurs événements indésirables hématologiques.

13. Ivaltinostat destiné à être utilisé selon la revendication 12, sachant que les événements indésirables hématologiques comprennent une neutropénie de grade 3 ou une thrombocytopénie de grade 3, et sachant que la quantité d'ivaltinostat est réduite à environ 80 % lorsqu'un deuxième épisode de neutropénie de grade 3 ou de thrombocytopénie de grade 3 survient, ou sachant que l'ivaltinostat est interrompu lorsqu'un troisième épisode de neutropénie de grade 3 ou de thrombocytopénie de grade 3 survient.

14. Ivaltinostat destiné à être utilisé selon la revendication 12, sachant que les événements indésirables hématologiques comprennent une neutropénie de grade 4, une thrombocytopénie de grade 4, ou une neutropénie fébrile de grade 3, et
sachant que la quantité d'ivaltinostat est réduite à environ 80 % lorsqu'un premier épisode de neutropénie de grade 4, de thrombocytopénie de grade 4, ou de neutropénie fébrile de grade 3 survient, ou
sachant que la quantité d'ivaltinostat est réduite à environ 64 % lorsqu'un deuxième épisode de neutropénie de grade 4, de thrombocytopénie de grade 4, ou de neutropénie fébrile de grade 3 survient, ou
sachant que l'ivaltinostat est interrompu lorsqu'un troisième épisode de neutropénie de grade 4, de thrombocytopénie de grade 4, ou de neutropénie fébrile de grade 3 survient.

15. Ivaltinostat destiné à être utilisé selon la revendication 12, sachant que les événements indésirables hématologiques comprennent une neutropénie fébrile de grade 4, et
sachant que la quantité d'ivaltinostat est réduite à environ 80 % lorsqu'un premier épisode de neutropénie fébrile de grade 4 survient, ou
sachant que l'ivaltinostat est interrompu lorsqu'un deuxième épisode de neutropénie fébrile de grade 4 survient.
